# EUROPEAN PATENT SPECIFICATION

(11) **EP 3 749 231 B1**
(45) Date of publication and mention of the grant of the patent: **07.08.2024**
(21) Application number: 18764370.5
(22) Date of filing: 08.03.2018
(51) Int. Cl.: A61B 17/34, A61B 1/313, A61B 17/00, A61B 1/00, A61B 1/04, A61B 1/06, A61B 17/02, A61B 17/24, A61B 17/70, A61B 90/00, A61B 1/12

(54) **DEVICES FOR PROVIDING SURGICAL ACCESS**
VORRICHTUNGEN ZUR BEREITSTELLUNG EINES CHIRURGISCHEN ZUGANGS
DISPOSITIFS POUR FOURNIR UN ACCÈS CHIRURGICAL

(30) Priority: 08.03.2017 US 201762468475 P; 18.10.2017 US 201715786858
(43) Date of publication of application: 16.12.2020
(73) Proprietor: Medos International Sàrl, 2400 Le Locle (CH)
(72) Inventor: WHITE, Michael, 4436 Oberdorf (CH); RICHTER, Joern, 4436 Oberdorf (CH); KLETT, Jan, 4436 Oberdorf (CH); GULLY, Stephane, 4436 Oberdorf (CH); ZOLLMAN, Veronique Christine, 4436 Oberdorf (CH); KOCHARIAN, Richard, Warsaw, Indiana 46581 (US)
(74) Representative: Carpmaels & Ransford LLP
(86) International application number: PCT/US2018/021454
(87) International publication number: WO 2018/165367

(56) References cited:
- WO-A1-2007/059068
- US-A- 5 618 293
- US-A1- 2005 137 461
- US-A1- 2006 200 186
- US-A1- 2008 242 930
- US-A1- 2009 259 184
- US-A1- 2010 268 241
- US-A1- 2013 304 106
- US-A1- 2014 378 985
- US-A1- 2015 087 913
- US-A1- 2016 256 036
- US-A1- 2018 098 789

## Description

### FIELD

The present application relates to devices and methods for providing surgical access, e.g., using adjustable length surgical access devices.

### BACKGROUND

Traditionally, incisions are made into a patient's body in order to access a patient's surgical region. Often, these incisions are made large enough to accommodate the insertion and removal of various implants, instruments and other objects. Such incisions provide unrestricted access through the patient's skin, and to the patient's anatomy at and around the surgical region. Such free access to and movement within the patient's body may provide certain advantages, but can increase the invasiveness of the procedure and can expose the patient to additional risks, for example, due to the various moving objects during surgery undesirably making contact with the patient, for example, at areas other than the surgical site.

To this end, access systems such as tubular access ports have been developed to provide enhanced targeting and accuracy to the patient's surgical region of interest. These ports can be used in minimally-invasive or percutaneous surgical procedures. For instance, microsurgical spinal bone resections and spinal decompressions, to name a few surgical procedures, are performed under microscopic view through openings in miniature percutaneous tubular access ports. Rather than inserting implants, instruments, objects, and even body parts (e.g., surgeon's hands) freely into the patient's body via the incision, the tubular access port is inserted through the incision and stabilized at or near the patient's surgical region, leaving a proximal end of the tubular access port accessible from above the patient's skin. Once inserted, the tubular access port provides a working channel from the patient's skin to the surgical site and protects and prevents trauma to, for example, bone, tissue, and nerves by objects being moved to and from the surgical site through the access port.

These tubular access ports are designed to have lengths capable of reaching the surgical region while being accessible from outside of the patient's skin or incision. Because the tubular access port is used for various surgeries, in order to access a number of surgical regions located at varying depths in the patient's body, traditional tubular access ports are manufactured in many sizes to achieve the desired surgical objective. Manufacturers of these access ports are thus tasked with developing and maintaining adequate supplies of a large variety of access tubes, each of which can have varying specifications (e.g., lengths). Adequate machinery and human supervision is required to oversee the manufacturing of a large and diverse portfolio of access ports. Likewise, traditional surgical centers such as hospitals are left with the burden of purchasing and storing a large and diverse inventory of access ports, in order to be prepared to use the adequate-length access port for any surgery at any given time. US2009/0259184A1 describes an apparatus which may serve as a surgical portal for the reception of surgical instruments for use in laparoscopic or similar surgery. The apparatus may include inner and outer tubular elongate members adapted for a longitudinal translation relative to one another to effect a length adjustment of the apparatus. An interface between the two elongate members includes a helical thread providing a mechanism for the translation of the elongate members, and indentations in one of the members provides a locking mechanism for affixing the length. US2006/0200186A1 describes an access device for providing access from a skin incision to a surgical site is described. The access device has a first section having a proximal end and a distal end defining a first path therethrough. The access device has a second section having a proximal end and a distal end defining a second path therethrough, the second section is movable relative to the first section, and the first and second sections cooperate to form a continuous path such that movement of the second section changes the length of the path. US2010/0268241A1 describes a telescoping access cannula comprising: an outer tube; an inner tube carried by the outer tube, the inner tube being coaxial with the outer tube and longitudinally movable relative to the outer tube; and a rotatable member carried by the outer tube and connected to the inner tube, wherein rotation of the rotatable member causes longitudinal movement of the inner tube relative to the outer tube.

### SUMMARY

The surgical access device according to the invention is defined in claim 1. Preferred embodiments are presented in the dependent claims. The surgical methods disclosed are not claimed but are useful for the understanding of the invention.

Further adjustable-length surgical access devices are disclosed herein, which can advantageously allow an overall length of the access device to be quickly and easily changed by the user. The access devices herein can reduce or eliminate the need to maintain an inventory of many different length access devices. In some embodiments, the length of the access device can be adjusted while the access device is inserted into the patient. This can reduce or eliminate the need to swap in and out several different access devices before arriving at an optimal length access device. This can also reduce or eliminate the need to change the access device that is inserted into a patient as the depth at which a surgical step is performed changes over the course of a procedure. Rather, the length of the access device can be adjusted in situ and on-the-fly as needed or desired to accommodate different surgical depths.

In some embodiments, an access device can include an upper tube having an opening formed therethrough, the upper tube including: a proximal end configured to prevent proximal movement of a spring, the spring forming an opening through which the upper tube is disposed; and one or more fingers formed at a distal end of the upper tube and extending toward a proximal end of the upper tube, the one or more fingers having ribs formed on an inner surface thereof; a middle tube having an opening formed therethrough for slidably receiving the upper tube, the middle tube including a proximal end configured to apply force on the spring; and a lower tube slidably disposed at least in part within the distal end of the upper tube and having an opening formed therethrough, the lower tube including grooves disposed on at least a portion of an outer surface of the lower tube and configured to engage with the ribs on the inner surface of the one or more fingers of the upper tube; wherein the middle tube is biased away from the upper tube in a configuration in which the spring is uncompressed, causing the distal end of the middle tube to compress the one or more fingers of the upper tube and lock the position of the lower tube by engaging the ribs on the inner surface of the fingers with the grooves on the outer surface of the lower tube, restricting the proximal and distal movement of the lower tube relative to the upper tube, and wherein the access device is actuated by proximally moving the middle tube relative to the upper tube against the bias of the spring, causing the one or more fingers to become exposed and move away from the lower tube, and enabling proximal and distal movement of the lower tube relative to the upper tube.

The openings formed through one or more of the upper tube, the middle tube and the lower tube can be round, square or rectangular openings. One or more of the upper tube, middle tube and lower tube can have round, square or rectangular shapes formed by their outer surfaces. To prevent the proximal movement of the spring, the proximal end of the upper tube can include a stop portion having an outer surface with a circumference larger than a circumference of an adjacent portion of the upper tube. The stop portion can be configured to prevent proximal movement of the spring relative to the distal end of the upper tube, and to cause the spring to compress when force is applied thereto in a proximal direction by the proximal end of the middle tube. The middle tube can include one or more handles extending away from an outer surface of the proximal end of the middle tube. The proximal movement of the middle tube can be caused by the application of force, in a proximal direction, on the one or more handles. The one or more fingers can be defined by slits formed through the outer and inner surfaces of the upper tube at its distal end. The grooves on the inner surface of the fingers of the upper tube can be configured to mate with the grooves on the outer surface of the lower tube, to restrict the proximal and distal movement of the lower tube relative to the upper tube. The size of the opening of the upper tube at its distal end when the fingers of the upper tube are compressed can be smaller than or equal to the size of the lower tube at its proximal end. The size of the opening of the upper tube at its distal end when the fingers of upper tube are flanged can be larger than the size lower tube at its proximal end. The size of the opening of the middle tube can be: (i) larger than the size of the upper tube at its distal end; (ii) larger than the size of the lower tube; and (iii) smaller than the size of the spring. A length of the access device can be adjustably set by the locked position of the lower tube in which its proximal and distal movement relative to the upper tube is restricted by the engagement of the grooves on the inner surface of the fingers with the grooves on the outer surface of the lower tube.

In some embodiments, a method for operating an access device can include identifying a target depth required to access a surgical site in a patient's body; adjusting the length of a surgical access device based on the identified target depth; and advancing a distal end of the surgical access device toward the surgical site in the patient's body.

Adjusting of the length of the surgical access device can include advancing the surgical access device from a biased configuration to an actuated configuration, to allow distal and proximal sliding of a lower tube of the access device relative to an upper tube of the access device; sliding the lower tube to a target position relative to the upper tube; and advancing the surgical access device from the actuated configuration to the biased configuration, to lock the target position of the lower tube relative to the upper tube. The advancing of the surgical access device from the biased configuration to the actuated configuration can include causing one or more fingers disposed on the distal end of the lower tube to flange by applying one or more of (i) distal force on the upper tube, and (ii) proximal force on the middle tube, against the bias of a spring positioned between a proximal end of the upper tube and a proximal end of the middle tube, causing the middle tube to slide proximally toward the proximal end of the upper tube, wherein, in the actuated configuration, the flanged fingers have an opening configured to enable the lower tube to slide distally and proximally therein. The advancing of the surgical access device from the actuated configuration to the biased configuration can include releasing the distal force on the upper tube or the proximal force on the middle tube, causing the spring to bias to its uncompressed configuration and driving the middle tube distally away from the proximal end of the upper tube, such that the fingers of the upper tube are compressed by the inner surface of the middle tube, and causing grooves disposed on an inner surface of the fingers of the upper tube to mate with grooves disposed on an outer surface of the lower tube to prevent the distal and proximal movement of the lower tube relative to the upper tube.

The method can include delivering a material to the surgical site through an opening formed through the surgical access device, the material including one or more of: (i) a flowable material selected from the group consisting of hemostat and gelatin, and (ii) a powdered material selected from the group consisting of powdered pig bladder, pixie dust, and pixie power.

In some embodiments, a surgical access device can include an inner tube; and an outer tube having a distal end and a proximal end, and a hole formed therethrough, the hole being configured to receive the inner tube, wherein an external surface of the inner tube generates friction with the internal surface of the outer tube, and wherein movement of the inner tube distally and proximally relative to the outer tube is restricted by the friction generated by the external surface of the inner tube with the internal surface of the outer tube.

In some embodiments, an access device can include a tube having an opening formed therethrough from a distal end to a proximal end of the tube, the tube comprising one or more sets of cut regions along an external surface of the tube for separating the tube into a useable portion and a disposable portion, wherein the useable portion has a length configured to surgically access an area of a patient's body.

The sets of cut regions can include one or more perforations. The sets of cut regions can be provided at fixed intervals along a portion of the length of the tube. Each of the sets of cut regions can be provided around the circumference of the external surface of the tube. Each of the sets of cut regions can be provided around less than the entire circumference of the external surface of the tube. Each of the perforations can penetrate through the tube from the external surface to the internal surface.

The access device can include a ring portion comprising two or more concentric walls extending from a proximal end to a distal end of the ring portion, the two or more concentric walls being separated by a space configured to receive the proximal end of the tube. One of the two or more concentric walls can include one or more teeth features angled facing the proximal end of the ring portion. The one or more teeth can be configured to penetrate the external surface of the proximal end of the tube when the ring portion is in a mounted configuration. The ring portion can be placed in the mounted configuration by (i) sliding the tube between two of the two or more concentric walls, one of which includes the one or more teeth features, from the distal end of the ring portion toward the proximal end of the ring portion, and (ii) pulling the tube from the proximal end of the ring portion toward the distal end of the ring portion, causing the penetration of the one or more teeth into the external surface of the tube. The one of the two or more walls including the one or more teeth features can have the one or more teeth features disposed on a surface facing another of the two or more concentric walls. The two or more concentric walls can include an exterior wall, an interior wall and a middle wall provided between the exterior wall and the interior wall. The middle wall can be the one of the two or more concentric walls that includes the one or more teeth features. The one or more teeth features can be provided on an internal surface of the middle wall.

In some embodiments, a method of operating an access device can include identifying a target depth required to access a surgical site in a patient's body; adjusting the length of a surgical access device based on the identified target depth; and advancing a distal end of the surgical access device toward the surgical site in the patient's body.

The adjusting the length of the surgical device can include removing a disposable portion of a tube of the surgical access device, by separating the disposable portion from the rest of the tube. The separating of the disposable portion can include twisting, bending or applying force against opposite sides of the tube of the surgical access device relative to a fixed pivot point. The fixed pivot point can be located at one of a set of cut regions disposed on the external surface of the tube. The set of cut regions can include one or more perforations. Each of the sets of cut regions can be provided around the circumference of the external surface of the tube. Each of the perforations can penetrate through the tube from the external surface to the internal surface.

The method can include mounting a tube of the adjusted-length surgical device onto a ring portion. The mounting of the tube onto the ring portion can include sliding the proximal end of the tube in a distal end of the ring portion, toward a proximal end of the ring portion, the proximal end of the tube being slid between two walls of the ring portion; pulling the tube in a direction from the proximal end of the ring portion toward the distal end of the ring portion, wherein the pulling of the tube causes the teeth features disposed on a surface of a first wall among the two walls to grip the tube by penetrating into an external surface of the tube, the teeth features being disposed on the surface of the first wall facing a second wall among the two walls. The method can include stabilizing the surgical access device advanced into the surgical site of the patient's body by connecting a stabilizing feature of the ring portion to a corresponding stabilizing feature on an anchor device.

In some embodiments, a surgical access device can include an inner tube having an opening formed therethrough and a knob protruding from an external surface of the inner tube; and an outer tube having an opening formed therethrough and one or more slots connected by a path, the one or more slots and the path penetrating through an external and internal surface of the outer tube and being operable to receive the knob of the inner tube, wherein rotating the inner tube in a first direction relative to the outer tube causes the inner tube to be positioned in an adjustable-length configuration, in which the knob of the inner tube is slidably disposed along the path of the outer tube, and wherein rotating the inner tube in a second direction opposite the first direction relative to the outer tube causes the inner tube to be positioned in a fixed-length configuration, in which the knob of the inner tube is fixedly engaged within one of the one or more slots of the outer tube.

A proximal end of the inner tube can include a handle, the handle being operable to control the rotating of the inner tube in the first and second directions. The opening of the outer tube can be configured to receive a first surgical tool. The outer tube can include a sub-tube protruding from the external surface thereof, the sub-tube being configured to receive a second surgical tool. The inner tube in the adjustable-length configuration can be advanced distally and proximally such that the knob slides along the path of the outer tube. Fixing the knob of the inner tube in each of the slots of the outer tube can cause the length of the surgical access device to be altered.

In some embodiments, a method for operating a surgical access device can include identifying a target depth required to access a surgical site in a patient's body; adjusting the length of a surgical access device based on the identified target depth; and advancing a distal end of the surgical access device toward the surgical site in the patient's body.

The adjusting the length of the surgical access device can include rotating an inner tube of the surgical access device in first direction relative to an outer tube of the surgical access device, to position a knob protruding from an external surface of the inner tube within a path formed along the length of an outer tube, the inner tube being slidably engaged within the outer tube; advancing the inner tube distally or proximally relative to the outer tube, such that the knob of the inner tube slides along the path of the outer tube; rotating the inner tube in a second direction opposite the first direction relative to the outer tube, to position the knob of the inner tube within one of a plurality of slots formed along the length of the outer tube and extending from the path, wherein the positioning of the knob of the inner tube within one of the plurality of slots causes the proximal and distal position of the inner tube relative to the outer tube to be fixed.

In some embodiments, a surgical access device can include an inner tube including one or more engagement features of a first type disposed on an outer surface thereof; and an outer tube telescopically mated with the inner tube, and including one or more engagement features of a second type formed at least on the inner surface thereof; wherein the first type of engagement features and the second type of engagement features are complementary to each other, and wherein a length of the surgical access device is adjusted by one of: (i) rotating the inner tube relative to the outer tube such that the one or more engagement features of the inner tube slide along the corresponding one or more engagement features of the outer tube, and (ii) applying opposite proximal or distal forces on the inner tube and outer tube, thereby causing engagement features of the inner tube to slip from the corresponding one or more engagement features of the outer tube to adjacent others of the one or more engagement feature of the outer tube.

The one or more engagement features of the inner tube and the outer tube can form oblique angles relative to a longitudinal axis of the inner tube and/or the outer tube. The one or more engagement features of the inner tube and the outer tube can be formed of a deformable material enabling their slipping therebetween. The one or more engagement features of the inner tube and the outer tube can be formed around part or all of the circumference of the inner tube and the outer tube, respectively.

In some embodiments, a surgical access device can include an inner tube having a distal end, a proximal end, and an opening formed therethrough, the inner tube comprising one or more ratchet features disposed along part of a length of an exterior surface of the inner tube, the one or more ratchet features comprising one or more tooth features; and an outer tube having a distal end, a proximal end, and an opening formed therethrough, the opening at the proximal end of the outer tube being configured to receive the distal end of the inner tube, and the outer tube comprising a lip feature formed around the circumference of the proximal end of the outer tube, the lip feature being configured to engage with one or more of the tooth features of the one or more ratchet features of the inner tube, wherein a length of the surgical access device is adjusted by applying opposite proximal and distal forces on the inner and outer tubes causing the lip feature of the outer tube to slip over one or more of the tooth features of the one or more ratchet features of the inner tube.

Engaging the lip feature of the outer tube with one or more of the tooth features of the inner tube can cause the distal and proximal movement of the inner and outer tube, relative to each other, to be restricted. The proximal end of the inner tube can be cone-shaped and can flange proximally and externally relative to the rest of the of the inner tube. The external surface of the cone shaped portion of the inner tube can be configured to contact a surface of skin of a patient's body when the surgical access device is inserted in the patient's body. A circumference of the internal surface of the outer tube at the lip feature can be smaller than a circumference of the external surface of the inner tube at the one or more tooth features.

The access device can include an auxiliary tool comprising a handle portion formed at a proximal end thereof, two or more prongs, and two or more tabs formed at corresponding distal ends of the two or more prongs, and the two or more prongs biased apart from one another, wherein the inner tube comprises one or more slots formed along part of the length of the inner tube, through the external and internal surfaces of the inner tube, wherein the outer tube comprises one or more slots formed along part of the length of the outer tube, through the external and internal surface of the outer tube, the one or more slots of the outer tube being shorter than the one or more slots of the inner tube, and wherein the auxiliary tool is engaged with the inner and outer tubes by squeezing the two or more prongs against their bias, proximally and distally moving the auxiliary tool along the opening of the inner tube, and releasing the two or more prongs causing their tabs to extend through the one or more slots of the inner tube and the one or more slots of the outer tube. The length of the surgical access device can be adjusted by proximally pulling or distally pushing the auxiliary tool when the tabs are extending through the one or more slots, causing distal or proximal force to be applied on the one or more slots of the outer tube and the distal and proximal position of the outer tube relative to the inner tube to change as the tabs slide along the one or more slots of the inner tube. The proximal pulling or the distal pushing of the auxiliary tool can cause the lip feature of the outer tube to slip over one or more of the tooth features of the inner tube.

In some embodiments, a method for adjusting a length of a surgical access device can include applying opposite distal and proximal forces on an inner tube and an outer tube of the surgical access device, the inner tube being slidably disposed within an opening of the outer tube, wherein the applying of the opposite distal and proximal forces causes a lip feature on an internal surface of a proximal end of the outer tube to slip past one or more tooth features of one or more ratchet features formed on an external surface of the inner tube; and terminating the opposite distal and proximal forces on the inner tube and the outer tube when the lip feature of the outer tube is engaged with one or more of the tooth features at a position in which the length of the surgical access device is equal to a desired length.

In some embodiments, a method for adjusting a length of a surgical access device can include applying distal or proximal force on an auxiliary tool positioned within an opening formed through an inner tube, the auxiliary tool comprising two or more prongs and or two or more corresponding tabs extending outwardly from distal ends of the two or more prongs through two or more slots formed through the inner tube and two or more slots formed through the outer tube, the two or more slots formed through the outer tube being shorter than the two or more slots formed through the inner tube, wherein applying the distal or proximal force on the auxiliary tool causes the two or more tabs to push or pull, respectively, against distal or proximal edges of the slots, such that the outer tube slides distally or proximally relative to the inner tube as the two or more tabs move distally or proximally along the one or more slots of the inner tube.

The applying the distal or proximal force on the auxiliary tool can further cause a lip feature formed on an internal surface of a proximal end of the outer tube to skip over one or more tooth features of one or more ratchet features formed on an external surface of the inner tube. The distal and proximal movement of the outer tube relative to the inner tube can be restricted when the lip feature of the outer tube is engaged with one or more of the tooth features of the inner tube. The auxiliary tool can be positioned within the opening of the inner tube by: inwardly squeezing the two or more prongs against their bias, such that the distance between the two or more prongs is less than the diameter of the opening of the inner tube; distally or proximally moving the auxiliary tool within the opening of the inner tube; and releasing the two or more prongs such that their bias causes the two or more tabs of the two or more prongs to separate and extend through the two or more slots formed in the inner tube and the two or more slots formed in the outer tube.

In some embodiments, a surgical access device can include a tube-shaped body having an external surface and an internal surface, the internal surface forming an opening through the surgical access device from a distal end to a proximal end; a plurality of longitudinal slits formed through the body, extending distally from the proximal end; and a plurality of bendable arms formed longitudinally through the body of the surgical access device, extending distally from the proximal end, each of the plurality of bendable arms being defined by two of the plurality of slits, wherein the plurality of bendable arms are configured to be externally bent relative to a pivot point to adjust the length of the access device.

The access device can include a ring wrapped around the external surface of the body, wherein the plurality of bendable arms are bent over a proximal end of the ring. The access device can include a stabilization material for fixing the position of the plurality of bendable arms on skin of a patient, the stabilization material comprising one or more of sutures, adhesive or tape. At least one of the plurality of bendable arms can engage with one or more posts having a fixed position relative to the body of a patient, the posts restricting the rotation of the one of the plurality of bendable arms.

In some embodiments, a method for adjusting a length of a surgical access device can include inserting a distal end of a surgical access device into a body of a patient, the surgical access device having a tube-shaped body; sliding a support ring distally along the body of the surgical access device such that a proximal end of the support ring is positioned above the surface of the skin of the patient; and bending one or more bendable arms against the proximal end of the support ring such that external surfaces of the one or more bendable arms are placed in contact with the skin of the patient, wherein the one or more bendable arms are formed longitudinally along the body of the surgical access device, extending distally from the proximal end of the surgical access device, the one or more bendable arms being separated by slits penetrating through the body of the surgical access device.

The one or more bendable arms can be secured to skin using a stabilization object including one or more of an adhesive, tape and sutures. At least one of the one or more bendable arms can be positioned between two or more posts having a fixed position relative to the body of the patient, such that rotation of the at least one of the one or more bendable arms is prevented.

In some embodiments, a surgical access device can include an inner tube having a distal end and a proximal end, a hole formed therethrough from the distal end to the proximal end, and one or more first ribbed features wrapped around the circumference of at least part of an external surface of the inner tube; and an outer tube having a distal end and a proximal end, a hole formed therethrough from the distal end to the proximal end, and one or more second ribbed features wrapped around the circumference of at least part of an internal surface of the outer tube, the outer tube having the inner tube slidably engaged therein, wherein the one or more first ribbed features are configured to engage with the one or more second ribbed features, and wherein a length of the surgical access device is adjusted by applying opposite distal and proximal forces on the inner and outer tubes such that at least one of the one or more first ribbed features slips over at least one of the one or more second ribbed features.

The distal and proximal positions of the inner and outer tube, relative to each other, can be fixed when the one or more first ribbed features are engaged with the one or more second ribbed features. The one or more first ribbed features and the one or more second ribbed features can be convex or concave structures. The access device can include a collar feature operable to connect to a stabilization object having a fixed position relative to a body of a patient or an obj ect.

In some embodiments, a method for adjusting the length of a surgical access device can include applying a force to a first tube of the surgical access device, the force being a distal or proximal force relative to a second tube of the surgical access device; causing one or more ribbed features of the first tube to slide past one or more ribbed features of the second tube; and releasing the force applied to the first tube when the first tube has reached a position, relative to the second tube, in which a length of the surgical access device is equal to a target length.

The first tube can be an inner tube having the one or more ribbed features formed on an external surface thereof. The second tube can be an outer tube having the one or more ribbed features formed on an internal surface thereof, the outer tube having the inner tube slidably engaged therein. The one or more ribbed features of the inner tube can be complimentary to the one or more ribbed features of the outer tube, such that distal and proximal movement of the inner and outer tubes relative to each other is prevented when the one or more ribbed features of the inner tube are engaged with the one or more ribbed features of the outer tube.

In some embodiments, a surgical access device can include a tube including a plurality of tube segments connected by interlocking snap features, the snap features including a protruding feature and a depression feature, wherein each of the plurality of tube segments includes at least one of a protruding feature and a depression feature, wherein at least one of the plurality of tube segment includes a protruding feature, and another of the plurality of tube segments adjacent to the one of the plurality of tube segments includes a depression feature, and wherein the one of the plurality of tube features and the adjacent tube feature are attached to one another by the protruding feature of the one tube segment being engaged with the depression feature of the adjacent tube segment.

One tube segment can be detachable from the adjacent tube segment by disengaging the protruding feature of the one tube segment from the depression feature of the adjacent tube segment. The protruding feature of the one tube segment can be disengaged from the depression feature of the adjacent tube segment by applying a force to the protruding feature in a direction away from the depression feature.

In some embodiments, a surgical access device can include a tube having a distal end and a proximal end, and corrugated features along a portion of a length of the tube, the corrugated features being configured to be pulled and pushed to extend and retract the length of the tube. The corrugated features can be provided adjacent to the proximal end of the tube.

In some embodiments, a surgical access device can include a first tube having a proximal end and a distal end, the first tube including fingers extending from the proximal end to the distal end; a second tube having a proximal end and a distal end, the second tube including fingers extending from the distal end to the proximal end, wherein the fingers of the first tube and the fingers of the second tube being are spaced part from each other, and wherein the fingers of the first tube are configured to engage with the fingers of the second tube to fix the position of the first tube relative to the second tube.

The size, shape and position of the fingers of the first tube can be equal to the size, shape and position of the fingers of the second tube. The fingers of the first tube can distally and proximally slide between the fingers of the second tube. The circumference of the first tube can be equal to the circumference of the second tube.

### BRIEF DESCRIPTION OF DRAWINGS

This disclosure will be more fully understood from the following detailed description taken in conjunction with the accompanying drawings, in which Fig. 9A-9E disclose an embodiment of the present invention:
FIG. 1 is a side view of one exemplary embodiment of a surgical access device, shown in a disassembled state;
FIG. 2A is a side view of the surgical access device of FIG. 1, shown in an assembled state;
FIG. 2B is a perspective view of the surgical access device of FIG. 1;
FIG. 2C is another perspective view of the surgical access device of FIG. 1;
FIG. 3A is side view of the surgical access device of FIG. 1 in a locked position;
FIG. 3B is side view of the surgical access device of FIG. 1 in an unlocked position;
FIG. 3C is side view of the surgical access device of FIG. 1, schematically illustrating a length adjustment of the access device;
FIG. 3D is side view of the surgical access device of FIG. 1 in a locked position;
FIG. 4 is a sectional side view of another exemplary embodiment of a surgical access device;
FIG. 5 is a perspective view of another exemplary embodiment of a surgical access device;
FIG. 6A is a perspective view of an exemplary tube of another exemplary embodiment of a surgical access device;
FIG. 6B is another perspective view the tube of FIG. 6A;
FIG. 6C is a top view of an exemplary embodiment of a ring of the surgical access device of FIG. 6A;
FIG. 6D is a perspective view of the ring of FIG. 6C;
FIG. 6E is a another perspective view of the ring of FIG. 6C;
FIG. 7A is a perspective view of another exemplary embodiment of a surgical access device;
FIG. 7B is another perspective view of the surgical access device of FIG. 7A;
FIG. 7C is a bottom view of the surgical access device of FIG. 7A;
FIG. 7D is another perspective view of the surgical access device of FIG. 7A;
FIG. 8 is a perspective view of another exemplary embodiment of a surgical access device;
FIG. 9A is a side view of another exemplary embodiment of a surgical access device;
FIG. 9B is a perspective view of the surgical access device of FIG. 9A;
FIG. 9C is another perspective view of the surgical access device of FIG. 9A;
FIG. 9D is another perspective view of the surgical access device of FIG. 9A;
FIG. 9E is an exploded perspective view of the surgical access device of FIG. 9A;
FIG. 10A is a perspective view of another exemplary embodiment of an access device, shown inserted through a skin surface of a patient and secured to a remote anchor point;
FIG. 10B is another perspective view of the access device of FIG. 10A;
FIG. 10C is another perspective view of the access device of FIG. 10A;
FIG. 11A is another perspective view of the access device of FIG. 10A in a placement state;
FIG. 11B is another perspective view of the access device of FIG. 11A in a support ring placement state;
FIG. 11C is another perspective view of the access device of FIG. 11A in a state of bending the bendable arms;
FIG. 11D is another perspective view of the access device of FIG. 11A in a state of attaching the bendable arms to the patient;
FIG. 11E is another perspective view of the access device of FIG. 11A in a state of attaching a bendable arm to a remote anchor point;
FIG. 12 is a sectional side view of another exemplary embodiment of a surgical access device;
FIG. 13A is a side view of another exemplary embodiment of a surgical access device, in a snapped-in configuration;
FIG. 13B is another side view of the surgical access device of FIG. 13A in an unsnapping configuration;
FIG. 13C is a sectional side view of a snap feature of the surgical access device of FIG. 13A;
FIG. 14 is a side view of another exemplary embodiment of a surgical access device;
FIG. 15A is a perspective view of another exemplary embodiment of a surgical access device;
FIG. 15B is a perspective view of an unattached tube of the surgical access device of FIG. 15A; and
FIG. 16 is a perspective view of an exemplary connection feature of a surgical access device.

### DETAILED DESCRIPTION

Adjustable-length surgical access devices are disclosed herein, which can advantageously allow an overall length of the access device to be quickly and easily changed by the user. The access devices herein can reduce or eliminate the need to maintain an inventory of many different length access devices. In some embodiments, the length of the access device can be adjusted while the access device is inserted into the patient. This can reduce or eliminate the need to swap in and out several different access devices before arriving at an optimal length access device. This can also reduce or eliminate the need to change the access device that is inserted into a patient as the depth at which a surgical step is performed changes over the course of a procedure. Rather, the length of the access device can be adjusted in situ and on-the-fly as needed or desired to accommodate different surgical depths.

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments. The features illustrated or described in connection with one exemplary embodiment may be combined with the features of other embodiments. Such modifications and variations are intended to be included within the scope of the present disclosure. Further, to the extent features or steps are described as being, for example, "first" or "second," such numerical ordering is generally arbitrary, and thus such numbering can be interchangeable.

The present disclosure includes some illustrations and descriptions that include prototypes or bench models. A person skilled in the art will recognize how to rely upon the present disclosure to integrate the techniques, devices, and methods provided for into a product, such as a consumer ready, warehouse-ready, or operating room ready surgical device.

A person skilled in the art will appreciate that the present disclosure has application in conventional endoscopic, minimally-invasive, and open surgical procedures.

Exemplary embodiments of the present disclosure provide surgical access devices, and more specifically, adjustable-length surgical access devices. The surgical access device can include one or more tubes or portions that can be manipulated to obtain a surgical access device of a desired length that is optimal or desirable to access a patient's surgical site of interest. Examples of such manipulations can include pulling, pushing, twisting, rotating, pivoting, folding, sliding, and/or combinations thereof. Engagement features provided on the surgical access device, including, for example, on inner and outer surfaces thereof, can prevent, limit or restrict the lengthening and shortening of the surgical access device in certain circumstances, such as when the manipulations described above are performed with minimal or incidental force, e.g., below an identified force threshold. Such features can include grooves, threads, slots, protrusions, depressions, lips, and the like. Thus, the features can limit or prevent inadvertent lengthening and shortening of the surgical access device once the surgical access device has been configured to its desired length. The surgical access device can be or can include an enclosed tubular structure, a slotted or bladed retractor, a cannula, and so forth.

The surgical access device can include a proximal end, proximate to a user of the surgical access device, and a distal end, proximate to the patient or the patient's surgical region. Formed through the surgical access device can be a hole or opening, extending from the proximal end to the distal end, defining a working channel through which implants, instruments, or other objects can be inserted to or removed from the patient's body.

The surgical access device can include a body, which can refer to the structure that makes up the external and internal surfaces of the surgical access device. The internal surface of the body can define the working channel through the surgical access device. The surgical access device can be round, square, rectangular, or any other shape, as deemed necessary or desirable to access a patient's surgical region. Likewise, the working channel formed through the surgical access device can be round, square, rectangular or any other shape, as deemed necessary or desirable to enable the insertion and removal of implants, instruments, or other objects for surgery.

The thickness of the body of the surgical access device, and other of its dimensions (e.g., diameter, inner circumference, outer circumference) can vary, so long as the aspects described herein enabling length adjustment are retained.

### FIRST EMBODIMENT

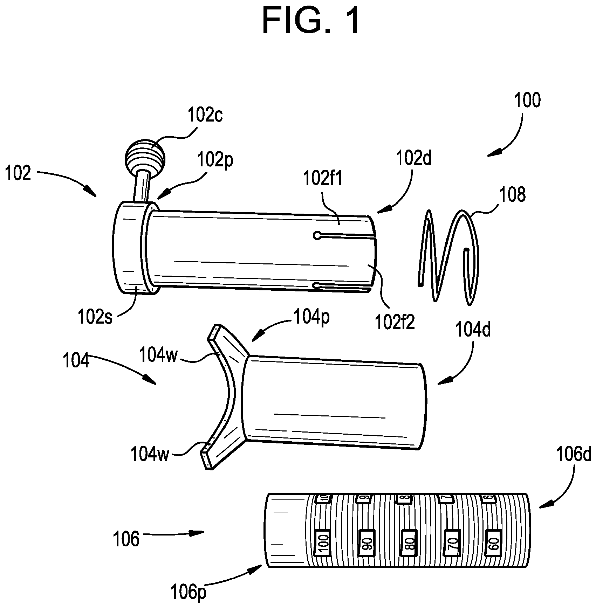

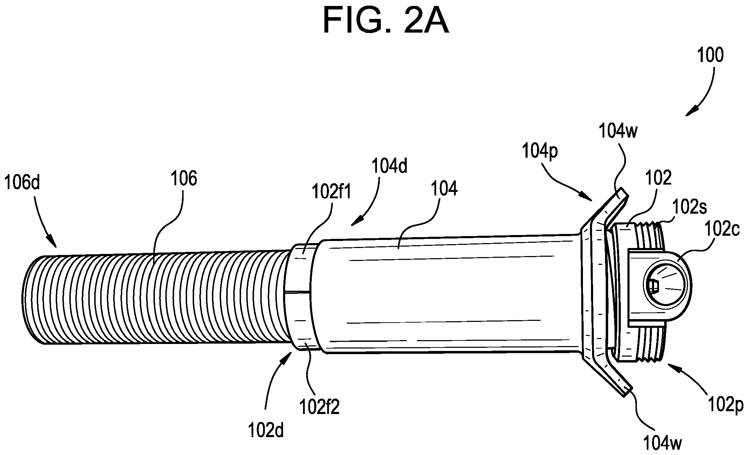

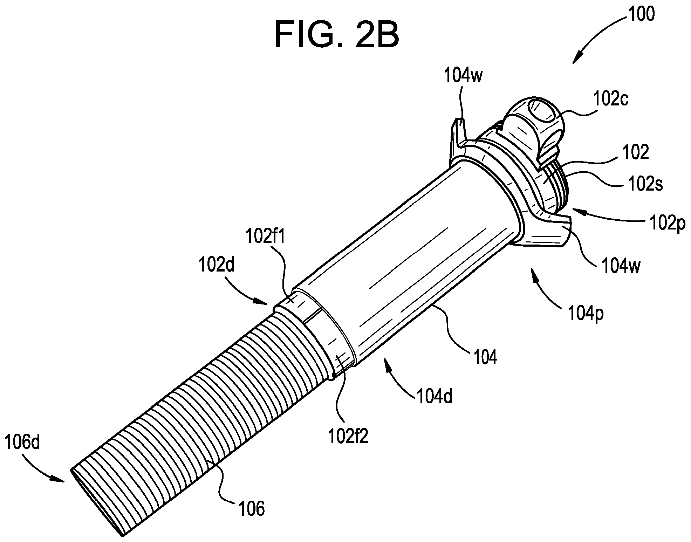

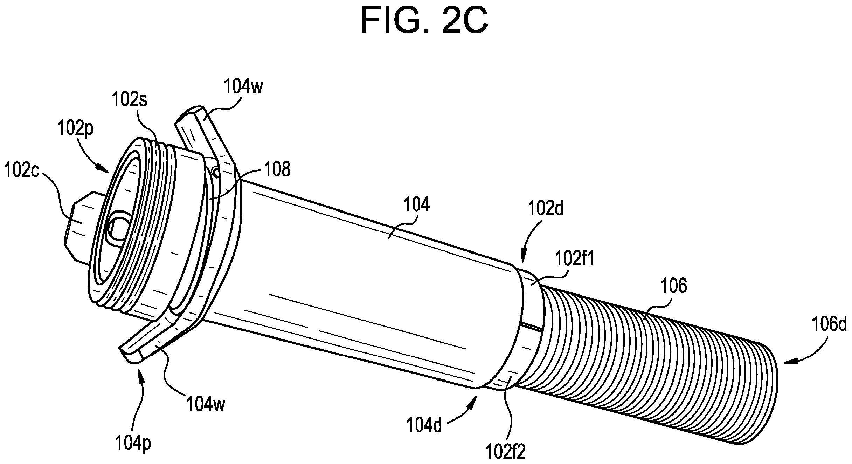

FIGs. 1, 2A to 2C, and 3A to 3D illustrate one exemplary embodiment of a surgical access device 100 configured to provide operative access to a patient's anatomy. Although the surgical access device 100 may be described with reference to its use to access a patient's intervertebral disc during spinal surgery, it should be understood that the surgical access device 100 can be configured and used in accordance with other surgical procedures to access to other surgical regions within a patient.

As shown, the surgical access device 100 can include an upper tube 102, a middle tube 104, a lower tube 106, and a spring 108. FIG. 1 illustrates the disassembled parts or components of the access device 100, namely the upper tube 102, middle tube 104, lower tube 106, and spring 108. In some embodiments, the access device 100 is referred to as a "telescoping tube," due to its being configured so that the upper, middle and lower tubes 102, 104, and 106 fit within one another in a way that allows them to slidably engage to enable the length of the access tube 100 to be extended and retracted.

The upper tube 102, middle tube 104, and lower tube 106 can be referred to as a first inner tube, outer tube, and second inner tube, respectively, to describe their inner and outer positions in the assembled surgical access device 100.

Still with reference to the disassembled access device 100 shown in FIG. 1, the upper tube 102, the middle tube 104, the lower tube 106, and the spring 108 can each include at least a portion that is substantially tubular or cylindrical. The upper tube 102, the middle tube 104, the lower tube 106, and the spring 108 can be assembled into a length-adjustable surgical access device 100 that can be used to access the patient's anatomy during surgery. To assemble the surgical access device 100, the distal end 102d of the upper tube 102 can be inserted through the spring 108. The distal end 102d of the upper tube 102, once inserted through the spring 108, can be inserted into the proximal end 104p of the middle tube 104. The proximal end 106p of the lower tube 106 can be inserted into or within the distal end 102d of the upper tube 102. In use, the middle tube 104 can be axially translated in a proximal direction relative to the upper tube 102, against the bias of the spring 108. With the middle tube 104 translated proximally, one or more resilient fingers 102f formed at the distal end of the upper tube 102 can expand radially-outward to disengage from the lower tube 106, thereby allowing the lower tube 106 to translate axially relative to the upper tube 102 to set an overall length of the access device 100. When set to the desired length, the middle tube 104 can be released, such that it returns distally under the bias of the spring 108. As the middle tube 104 moves distally, it can urge the fingers 102f radially-inward into engagement with the lower tube 106 to fix the length of the access device 100.

The upper tube 102 can include a proximal end 102p. The proximal end 102p can include or can have formed thereon a stop 102s configured to abut the proximal end 104p of the middle tube 104, to stop or prevent the middle tube 104 from excessive proximal movement relative to the upper tube 102. The stop 102s can extend radially about the proximal end 102p of the upper tube 102. In other words, to prevent excessive proximal movement of the middle tube 104, the outer surface of the stop 102s can have a circumference that is larger than (1) the circumference of the outer surface of the adjacent proximal end 102p of the upper tube 102, and (2) the circumference of the inner surface of the proximal end 104p of the middle tube 104. The outer surface of the stop 102s can be textured with grooves and/or ridges to enhance its gripping qualities, thereby allowing the access device 100 to be more easily manipulated by a user, for example, to stabilize or twist the access device 100.

The stop 102s can include a connector interface 102c which can allow the position and/or orientation of the access device 100 to be fixed via a connector or other support (not illustrated). The connector interface 102c can be built in to or can protrude outwardly from the stop 102s. A connector can be used to connect the connector interface 102c of the access device 100 to an ipsilateral or contralateral pedicle anchor or anatomical support, operating table, or other support or stabilizing feature (not illustrated). The connector interface 102c can be used as a stabilizer portion during actuation of the access device 100.

The stop 102s and/or the connector interface 102c can be formed integrally with the upper tube 102p, or can be manufactured as a separate component that is attachable to the upper tube 102p.

The upper tube 102 can include a distal end 102d. The distal end 102d can have formed thereon flexible fingers 102f1 and 102f2 (collectively "102f") configured to engage with the lower tube 106 and limit or prevent the distal or proximal movement of the lower tube 106 relative to the upper tube 102. The flexible fingers 102f can be formed by slits (or cuts) formed in the body of the upper tube 102, starting from the distal end 102d toward the proximal end 102p. The proximal end of the slits can include a circle or hole of larger diameter than the slits, to enable the fingers to more easily, and/or more widely, expand or deflect as explained below. The flexible fingers 102f can include any number of fingers and can be created by one or more slits.

The length of the slits on the body of the upper tube 102 can be the same or can vary. The length of the slits forming the fingers 102f can be determined based on the length of the middle tube 104 and/or the lower tube 106, on the amount of engagement that is desired between the upper tube 102 and the lower tube 106, and/or on the amount of deflection or expansion of the fingers 102f that is desired or to be allowed. The length of the slits forming the fingers 102f can be equal to or greater than the difference in length between the upper tube 102 and the middle tube 104. The length of the slits forming the fingers 102f can be equal to or greater than the length of the outer surface of the upper tube 102 at the distal end 102d that is exposed when the middle tube 104 is fully retracted. The length of the slits can be in the range of 1% to 33% of the length of the upper tube 102 (e.g., 1%, 5%, 10%, 15%, 20%, 25%, 30%, 33%).

The inner surface of the upper tube 102 can include grooves or ridges that are congruent with, complementary to, or that mate with counterpart grooves or ridges on the outer surface of the lower tube 106. The ridges or grooves can be spiral, circular or otherwise shaped to engage with counterpart features to limit or prevent movement of the upper and lower tubes 102 and 106, relative to one another, in a lengthwise, proximal and distal direction. The grooves or ridges on the inner surface of the upper tube 102 can be formed on the distal end 102d, e.g., on the fingers 102f. The grooves and/or ridges can be referred to as complementary engagement features.

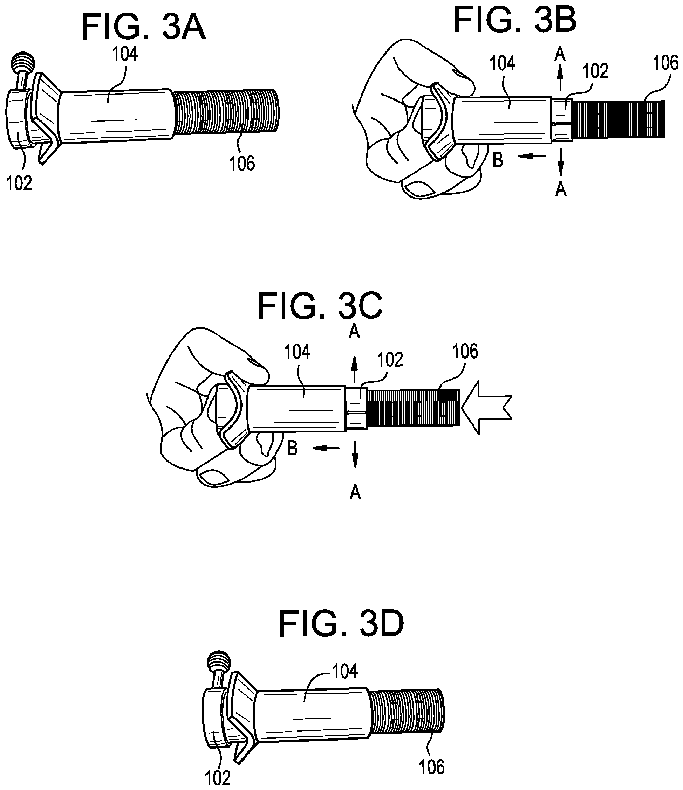

As explained in further detail below with reference to FIGs. 3A to 3D, the fingers 102f can be actuated (e.g., expand, deflect, flare) by proximally retracting the middle tube 104 relative to the upper tube 102, allowing the fingers 102f to expand or flare outward in the direction A illustrated in FIGs. 3B and 3C, thereby increasing or enlarging the diameter of the opening of the upper tube 102 at the distal end 102d where the fingers 102f are formed. When the fingers are in an expanded state (e.g., flared), as opposed to their default or rest state (e.g., contracted), the increased diameter of the distal end 102d can allow the lower tube 106 to slide or be moved distally and proximally relative to the upper tube 102. A desired total length of the access device 100 can be set by releasing the middle tube 104 from its retracted position, causing the middle tube 104 to move distally relative to the upper tube 102 as a result of the bias of the spring 108, and causing the fingers 102f to retract (e.g., opposite of direction A) and the diameter of the opening of the distal end 102d to decrease.

As mentioned above and described in further detail below, when the fingers 102f are flexed inward from their default position, the grooves on the inner surface of the upper tube 102 can engage with grooves in the outer surface of the lower tube 106. The lower tube 106 can include a distal end 106d and a proximal end 106p. The diameter of the opening of the tube 106 formed by the inner surface can be the same at the distal end 106d and at the proximal end 106p. The external surface of the lower tube 106 can include or have formed thereon grooves (or ridges) that are configured to engage with matching, mating, or complementary grooves on the internal surface of the upper tube 102. The grooves on the lower tube 106 can be provided at various regions along the length of the tube, as needed to achieve the desired length and stability characteristics of the access device 100 for its use in surgery.

The outer surface of the lower tube 106 can have markings, writing and/or other indications thereon. Markings provided along the length of the outer surface of the lower tube 106 can indicate lengths or other measurements (e.g., of the access device 100, of the lower tube 106, etc.) when the upper tube 102 is fixedly engaged with the lower tube 106. For example, the length of the access device 100 can be the measurement marked on the external surface of the lower tube 106 at the position where the distal end 102d of the upper tube 102 extends to.

As mentioned above and described in further detail below, to fix the position of the lower tube 106 relative to the upper tube 102 along a distal and proximal direction, and/or to fix the length of the access device 100, the middle tube 104 can be operated by proximal retraction and distal extension movements. The middle tube 104 can include a proximal end 104p and a distal end 104d.

The proximal end 104p of the middle tube 104 can include or can have formed thereon wings 104w (or rings, handles, ears, or another similar structure) that allow a user of the access device 100 to retract, pull or otherwise move or slide the middle tube 104 toward the proximal end 102p of the upper tube 102. The wings 104w can protrude or can be formed away from the external surface of the middle tube 104. The wings 104w or a portion thereof can extend away from the external surface of the middle tube 104 such that the wings 104w form a right angle or an oblique angle with the outer surface of the middle tube 104. The wings 104w can form a curve away from the external surface of the middle tube 104, such that the distal-most end of the wings 104w are formed away from the distal end 104d of the middle tube 104.

The dimensions, shape and other attributes of the wings 104w can vary as needed to provide an area where the user's fingers (e.g., middle and pointer fingers) can engage and pull the middle tube 104 toward the proximal end 102p of the upper tube 102. For instance, the curvature and length of the wings 104w can be determined and/or configured according to the average or maximum size of a human's index and/or middle fingers. Likewise, other configurations of the wings 104w, such as rings, can be rings that are formed external to or protruding from the outer surface of the middle tube 104 and that have an opening with a circumference larger than the average or maximum size of a human's index and/or middle fingers. It should be understood that the access device 100 can include one or more wings 104w.

As described in further detail below, when the middle tube 104 is retracted or pulled toward the proximal end 102p of the upper tube 102, the spring 108 that is disposed between the middle tube 104 and the upper tube 102 can be compressed. The size (compressed and uncompressed) and amount of force needed to compress the spring 108 can be determined based on various factors including the typical user's preference, the amount of time and speed at which the spring decompresses from the compressed state, the length of the tubes 102, 104, and 106, the material of the spring 108, and/or the thickness of the spring 108. The length of the spring 108 or the amount of lengthwise space that the uncompressed spring 108 covers when engaged with the upper tube 102 can be less than or equal to the difference in the length of the upper tube 102 and the middle tube 104. The spring 108 can be configured such that the amount of force needed to compress the spring 108 is (1) high enough so that the spring 108 returns towards a resting state to drive the middle tube 104 distally relative to the proximal end 102p of the upper tube 102 and maintain the set length of the access device 100; and (2) low enough so that the driving of the middle tube 104 distally relative to the proximal end 102p of the upper tube 102 is slow and controlled enough to not significantly alter the position of the access device 100 (e.g., relative to a patient) or the position of the tubes 102, 104, and 106 relative to one another.

As described above, the access device 100 can provide access to a patient's anatomy during surgery. Accordingly, the length of the access device 100 at its longest configuration can be large enough to reach an anatomical point of interest. To this end, each of the tubes 102, 104, and 106 can have a respective length such that, when the tubes 102, 104, and 106 are assembled together with the spring 108 to form the access device 100, the desired total length of the access device 100 can be achieved. The length of the upper tube 102 can be greater than the length of the middle tube 104 and the lower tube 106. The length of the middle tube 104 can be less than the length of the upper tube 102 and the lower tube 106. The length of the lower tube 106 can be greater than the length of the middle tube 104, and less than, equal to or greater than the length of the upper tube 102. The length of the lower tube 106 and/or the length of the grooved portion of the outer surface of the lower tube 106 can be equal to or greater than the distance from a patient's skin (e.g., where an incision is made and the access device 100 inserted) to the part or area of the patient's anatomy to which access is required, such that when the lower tube 106 is the only one of the tubes 102, 104, and 106 that is inserted into the incision, the lower tube 106 can reach and access that part or area of the patient's anatomy.

The openings or working channel created by the inner surfaces of the tubes 102, 104, and 106 can be large enough to enable objects (e.g., implants, instruments, or other objects) to be moved therethrough, to and from the distal end 100d and the proximal end 100p of the access device 100. The diameter and/or circumference of the opening of each of the tubes 102, 104, and/or 106 can vary from their distal ends to their proximal ends For example, the upper tube 102 can be configured such that the diameter or circumference of the opening at the proximal end 102p is larger than the diameter or circumference, respectively, of the opening at the distal end 102d. The openings of the tubes 102, 104, and/or 106 can gradually narrow or expand along their lengths.

The circumference of the opening of the middle tube 104 (at the proximal end 104p and the distal end 104d) can be larger than the circumference of the outer surface of the upper tube 102 (at the distal end 102d and the proximal end 102p), such that the distal end 102d of the upper tube 102 can be inserted through the opening at the proximal end 104p of the middle tube 104 and through the entirety of the length of the middle tube 104, exiting at the distal end 104d. The circumference of the opening of the middle tube 104 can be larger than the circumference of the outer surface of the lower tube 106. The circumference of the opening of the upper tube 102 at the distal end 102d and/or along the length of the fingers 102f, when the fingers 102f are in a radially-inward position, can be equal to or smaller than the circumference of the outer surface of the lower tube 106; and the circumference of the opening of the upper tube 102 at the distal end 102d and/or along at least a portion of the length of the fingers 102f, when the fingers 102f are at an expanded or radially-outward position, can be larger than the circumference of the outer surface of the lower tube 106. The circumference of the opening of the spring 108 can be larger than the circumference of the outer surface of the upper tube 102.

As described above, the tubes 102, 104, and 106, and the spring 108 can be assembled to form the access device 100. FIGs. 3A to 3D illustrate a process for using and/or adjusting the length of the access device 100 according to an exemplary embodiment. As will be described, the access device 100 can be actuated by (1) retracting the middle tube 104 relative to the upper tube 102, similar to a syringe; (2) adjusting the exposed length of the lower tube 106; and (3) releasing the middle tube 104.

More specifically, FIG. 3A illustrates the access device 100 in a default or locked state in which the length of the access device is fixed. When the access device 100 is in its default state, the spring 108 (not shown in FIG. 3A) can urge the middle tube 104 distally relative to the upper tube 102. That is, the proximal end 104p of the middle tube 104 can be forced to its furthest position from the proximal end 102p of the upper tube 102 by the bias of the spring 108. In this position, the internal surface of the middle tube 104 can maintain the fingers 102f of the upper tube 102 in a radially-inward position in which they engage with the grooves or other engagement features of the lower tube 106. In such a position of the middle tube 104, a small part (e.g., 1%, 2%, 5%, 10%, 15%) of the fingers 102f can be exposed so long as the fingers 102f are not free to expand out of engagement with the lower tube 106.

The access device 100 can be actuated by pulling, retracting or otherwise proximally moving or sliding, relative to the upper tube 102, in the direction B, the middle tube 104 toward the proximal end 102p of the upper tube 102, as shown in FIG. 3B. The actuation of the access device 100 can be performed by applying a pushing pressure or force on the proximal end 102p (or stop 102s) of the upper tube 102 (e.g., by a user's thumb), and applying an opposite pulling pressure or force on the wings 104w of the middle tube 104 (e.g., by the user's index and middle finger), against the bias of the spring 108.

When the middle tube 104 is in the retracted position, as shown in FIGs. 3B and 3C, the distal end 104d of the middle tube 104 is moved toward the proximal end 102p of the upper tube 102. Because the upper tube 102 is longer than the middle tube 104, such a proximal retraction of the middle tube 104 can cause the fingers 102f of the upper tube 102 to become exposed. When the fingers 102f are exposed and no longer compressed by the inner surface of the middle tube 104, the fingers 102f can be free to expand in the direction A. The flaring or expanding of the fingers 102f can cause the diameter of the opening at the distal end of the upper tube 102 and/or the circumference of the inner surface of the upper tube at the fingers 102f to increase in size (e.g., to a size larger than the diameter or circumference of the lower tube 106). This can cause the fingers 102f and the lower tube 106 to no longer be engaged or fixedly positioned, thereby allowing the lower tube 106 to be slid or translated proximally or distally relative to the distal end 102d of the upper tube 102, as shown in FIG. 3C.

The lower tube 106 can continue to be moved proximally and distally so long as the access device 100 is actuated (e.g., the middle tube 104 remains in the retracted position). When the lower tube 106 is moved to its desired location such that the access device 100 has a resulting optimal or desired length for surgical use, the proximal or pulling force on the middle tube 104 and/or the distal or pushing force on the upper tube 102 can be released, causing the upper tube 102 and the middle tube 104 to return towards their default positions, and the fingers 102f to be compressed by the internal surface of the middle tube 104, such that the position of the lower tube 106 relative to the upper tube 102 becomes fixed. In other words, as shown in FIG. 3D, when the force on the upper tube 102 and/or the middle tube 104 is released, the spring 108 can expand, causing the middle tube 104 to move distally, away from the proximal end 102p of the upper tube 102. In addition, the previously exposed fingers 102f can become unexposed by sliding within the inner surface of the distal end 104d of the middle tube 104. As a result, the fingers 102f can compress, causing the ridges or grooves on the inner surface of the fingers 102f to engage with the ridges or grooves on the outer surface of the lower tube 106, thereby locking or preventing the distal or proximal movement of the lower tube 106 relative to the upper tube 102. The resulting access device 100 can have a length that is determined based on the selected amount of exposed area of the lower tube 106, as set by the position where the lower tube 106 is locked. The access device 100 can then be used to access a target or desired area of the patient's anatomy located at a distance that is reachable by the access device 100 having been set at a desired length.

### SECOND EMBODIMENT

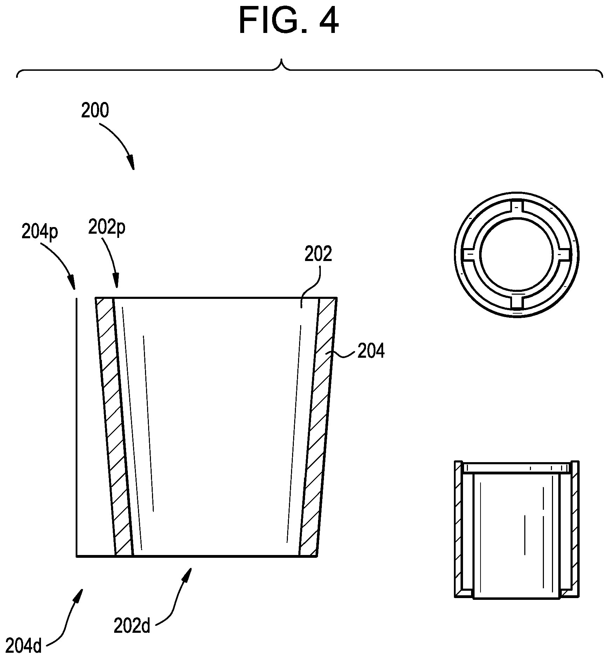

FIG. 4 illustrates another exemplary embodiment of a surgical access device 200. The surgical access device 200 can include an inner tube 202 and an outer tube 204. The inner tube and the outer tube can have respective proximal ends (202p, 204p) and distal ends (202d, 204d), and holes or openings formed therethrough from their distal ends to the proximal ends to define a working channel through the access device.

The inner tube 202 can be slidably disposed within the outer tube 204 - e.g., within the opening of the outer tube 204 to define a telescoping access device. The inner tube 202 and the outer tube 204 can be configured such that sufficient friction is created between the external surface of the inner tube 202 and the internal surface of the outer tube 204 to maintain a relative longitudinal position of the inner and outer tubes. To accomplish the desired friction between the tubes 202 and 204, the inner tube 202 can have an external surface with a circumference equal to or substantially equal to the circumference of the opening or internal surface of the outer tube 204.

To adjust the length of the surgical access device 200, opposite pulling and pushing forces can be applied to the tubes 202 and 204 to overcome the frictional force and axially translate the tubes relative to one another to shorten or lengthen the access device. When the pulling and pushing force is ceased, the friction between the tubes 202 and 204 can be effective to restrict the proximal and distal movement of the tubes 202 and 204 and to maintain the device 200 at the adjusted length. The inner and outer tubes 202, 204 can be rotationally fixed relative to one another, e.g., such that they inner and outer tubes cannot rotated with respect to one another about a central longitudinal axis of the device 200. The tubes 202, 204 can be rotationally fixed in various ways, such as by engagement between counterpart longitudinal rails and grooves, a pin-in-slot arrangement, or the like.

### THIRD EMBODIMENT

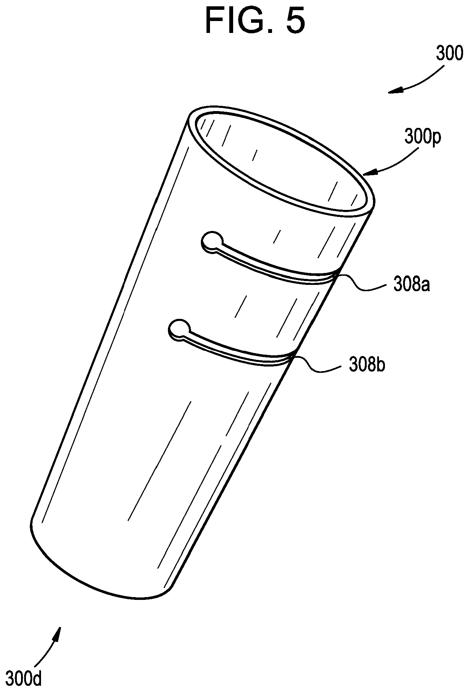

FIG. 5 illustrates another exemplary embodiment of a surgical access device 300 configured to provide operative access to a patient's anatomy. The surgical access device 300 can include a tube or body having an opening or working channel formed therethrough from a distal end 300d to a proximal end 300p. The working channel can be defined by an inner surface of the surgical access device 300. The working channel can be round, square, rectangular, and/or various other shapes, and can have a diameter or circumference of any size, so long as objects (e.g., implants, instruments, or other objects) can be moved to and from the proximal end 300p and the distal end 300d. The shape, diameter, and/or circumference of the working channel can change between the distal end 300d and the proximal end 300p. In other words, for example, the opening at the distal end 300d can be smaller or narrower than the opening at the proximal end 300p of the surgical access device 300. It should be understood that the shape of the access device 300, which is defined by its external surface, can match the shape of the opening (e.g., circle, rectangle, square), or can have a shape that is different than that of the opening.

The surgical access device 300 can be manufactured at a single maximum length determined to reach the deepest area of interest of a patient's body to be accessed during surgery. However, to enable the surgical access device 300 to be used at various lengths as needed to optimally or desirable access shallower regions in a patient's body, the access device 300 can include one or more slits or perforations 308 around at least part of the circumference of the external surface of the access device 300, which facilitate the breaking (e.g., splitting, tearing, separating) of the access device 300 into multiple parts. The perforations 308 can have regions (e.g., circular perforations) that are larger in diameter than the rest of the slits of the perforations, to provide for easier breaking thereat. Breaking the access device 300 into multiple parts can be achieved in various ways, such as by twisting the access device 300 at opposite sides of the selected perforation 308, or bending, folding or applying force to the access device at opposite sides of the selected perforation 308, e.g., about a fixed pivot point equal to the position of the selected perforation 308 on the access device 300.

As shown in FIG. 5, the access device 300 can include a plurality of perforations 308a and 308b formed around part of the circumference of the access device at various positions along the length of the access device 300. Although only two perforations (308a and 308b) are illustrated in FIG. 5, any number of perforations can be provided on the access device 300. The perforations can be provided at regular or irregular intervals along the length of the access device 300. The perforations can be provided starting at the distal end or at the proximal end of the access device 300.

The perforations 308 can be provided around less than the entire circumference of the access device 300 (e.g., 10%, 25%, 33%, 50%, 75%). The perforations can be provided around a part of the circumference of the access device 300 that sufficient to allow for manual breakage of the access device 300 into multiple parts, preferably without any machine or tool assistance.

The length, depth, and width of the perforations 308 can vary between individual perforations on the access device 300, or between different access devices, as deemed necessary or desirable to enable manual breakage. For instance, the length, depth, and width of the perforations 308 can be based on the properties of the material used to manufacture the access device 300, the thickness of the body of the access device 300, the circumference of the access device 300, or other parameters.

The perforations 308 can have a length or other measurement associated therewith and marked on the external surface of the access device 300. These markings can allow a user to identify the resulting length of the access device 300 when the access device is broken at the respective perforation 308. The access device 300 can be configured such that, when it is broken into multiple parts, either the distal end or the proximal end of the original full-length access device 300 can be the resulting access device 300 to be used to access a part of a patient's body.

The access device 300 can be provided without perforations, such that the access device 300 is instead cut at a desired region or length using a machine or tool. The access device 300 can include lines or markings indicating different measurements or lengths at which the access device can be cut using a machine or tool, thereby enabling identification of the resulting length of the access device 300 after it is cut into multiple parts.

### FOURTH EMBODIMENT

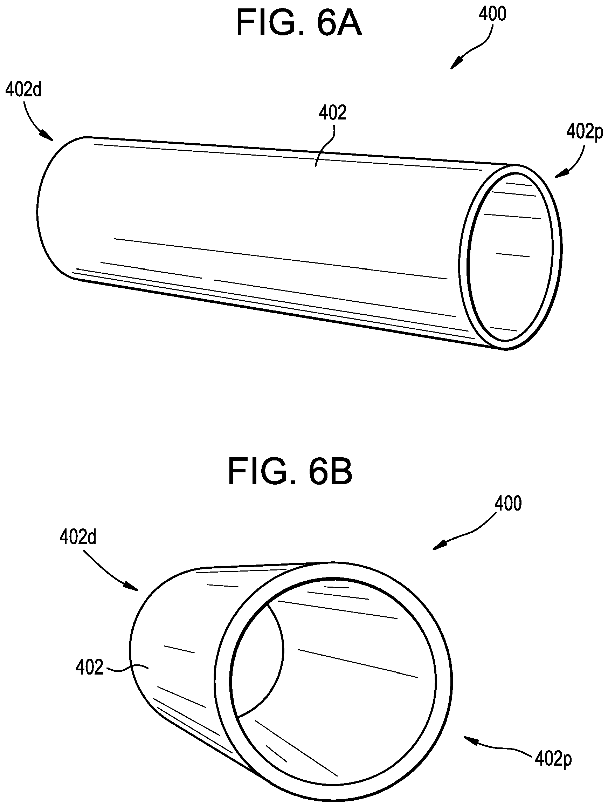

FIGs. 6A to 6E illustrate another exemplary embodiment of a surgical access device 400. As illustrated, the access device 400 can include a main body or tube 402 and a ring portion 404. The main body 402 can be cut, torn, or broken to the desired length, and the ring portion 404 can be attached thereto, e.g., to provide an anchoring point for stabilizing the main body or to cover the broken end of the main body. The tube 402 can be the access device 300 described above, for example, after having been split or cut into multiple parts to obtain a tube of a resulting optimal or desirable size for accessing a patient's target area.

As shown in FIGs. 6A to 6E, the tube 402 can include an opening or working channel formed therethrough, from a proximal end 402p to the distal end 402d. The shape and size of the opening, defined by the inner surfaces of the tube 402, can vary, as described herein. The shape and size of the opening can vary throughout the length of the tube 402. For example, the size of the opening can narrow from the proximal end 402p to the distal end 402d. The shape and size of the access device 400, as defined by its external surfaces, can also vary as described herein.

The ring portion 404 of the access device 400 can be attached or mounted onto the proximal end 402p of the tube 402. The proximal end 402p of the tube 402 can be the cut end of a tube after being cut or divided as described above in connection with FIG. 5. The ring 404, when attached to the tube 402, can provide enhanced handling and stabilizing of the access device 400 as explained in further detail below. The ring 404 can include a proximal side 404p and a distal side 404d. The ring 404 can include one or more concentric walls, e.g., an exterior wall 404e, an interior wall 404i, and a middle wall 404m as shown. The exterior, interior and middle walls 404e, 404i, and 404m can extend distally from the proximal end 404p, lengthwise, to a length deemed sufficient to stabilize the tube 402 and the ring 404 when they are engaged. The length of the walls 404e, 404i and/or 404m can be based on the length of the tube 402. That is, the walls 404e, 404i and 404m can have a length proportional to the length of the tube 402. The length of the walls 404e, 404i, and 404m can vary in size between each other.

The proximal side 404p of the ring 404 can be covered or sealed between the external wall 404e and the internal wall 404, such that the spaces between the walls 404e, 404i and 404m cannot be accessed from a proximal-to-distal direction. The part of the proximal side 404p that is internal or within the internal wall 404i can be open, such that an opening is created through the proximal end 404p to the distal end 404d of the ring 404. The distal side 404d of the ring 404 can be exposed, allowing the spaces between the walls 404e, 404i, and 404m to be accessed or entered from a distal-to-proximal direction, for example, to mount the ring 404 onto the tube 402 as explained in further detail below.

The interior wall 404i can have a smaller circumference than the middle wall 404m and the exterior wall 404e. The middle wall 404m can have a circumference smaller than that of the exterior wall 404e and larger than that of the interior wall 404i. The difference in size between the circumference of the middle wall 404m and the interior wall 404i - i.e., the gap therebetween -can be configured based on the thickness of the tube 402 at its proximal end.

The middle wall 404m can be a fully-closed circle. In other embodiments, such as that shown in FIGs. 6A to 6E, the middle wall 404m can be made up of or can include intermittent or spaced apart wall portions (e.g., 404m-1, 404m-2, etc.) that extend distally from the underside of the ring 404, and that form the wall 404m. The wall portions can be referred to as "fingers" that extend distally from the distal-facing side of the proximal end 404p of the ring 404. The wall portions of the wall 404m can be flexible and/or resilient such that their distal ends can move radially-inward or radially-outward. For example, the wall portions can flex or deflect away from the interior wall 404i and toward the external wall 404e when pressure is applied thereto as a tube 402 is inserted into the space between the middle wall 404m and the internal wall 404i. Such flexibility of the wall portions can enable insertion of a tube 402 having a thickness equal to or larger than the space between the middle wall 404m and the interior wall 404i, when the wall portions are not deflected from their resting position, to be inserted therebetween.

An internal surface of the middle wall 404m (e.g., the surface of the middle wall 404m facing the internal wall 404i), and or an outer surface of the internal wall can include one or more slits, teeth, or other surface features for engaging the tube 402. The surface features can be directional, e.g., unidirectional, such that the surface features allow insertion of the tube 402 in a proximal direction but resist or prevent removal of the tube 402 in a distal direction. For example, the surface features can include teeth that are angled facing toward the proximal side 404p of the ring 404. In this way, when the tube 402 is inserted from the distal side 404d of the ring 404 toward its proximal side 404p, along the space between the middle wall 404m and the internal wall 404i, the distal-most ends of the teeth of the middle wall 404 can contact and slide along the external surface of the proximal end 402p of the tube 402. In turn, pulling the tube 402 in a distal direction away from the ring 404 can cause the teeth of the wall 404m to penetrate the body of the tube 402, thereby creating or increasing a grip of the ring 404 on the tube 402.

The space between the middle wall 404m and the internal wall 404i can be measured from the distal end of the teeth of the middle wall 404m to the external surface of the internal wall 404i. In some embodiments, the ring 404 can include only an external wall 404e and an internal wall 404i, without a middle wall 404m. In such embodiments, the teeth of the middle wall 404m described above can be provided in the internal surface of the external wall 404e, and the space between the external wall 404e and the internal wall 404i can be configured similar to the space between the middle wall 404m and the internal wall 404i described above, to receive the proximal end 402p of the tube 402.

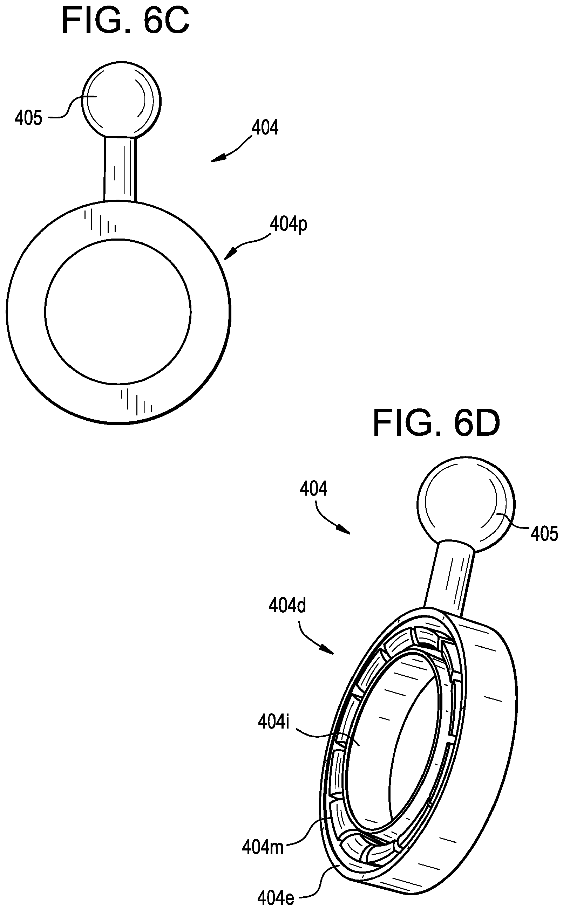

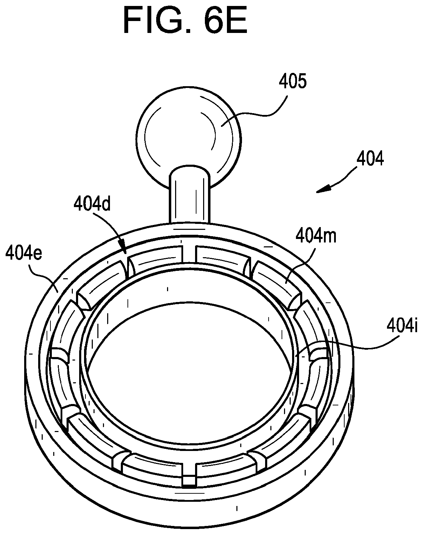

With the ring 404 mounted on the tube 402, the access device 400 can be more easily gripped by virtue of the larger size of the ring 404 compared to the tube 402. Moreover, the access device 400 can be stabilized using a stabilization feature 405 provided on the ring 404. The stabilization feature 405 can be a structure extending away from the external-facing side of the external wall 404e, and can be shaped in various ways. The stabilization feature 405 can be used to connect or attach the access device 400 to an ipsilateral or contralateral pedicle anchor or anatomical support, to a connector, operating table, or other support feature, which can provide stabilization of the access device. As shown in FIG. 6, the stabilization feature 405 can be shaped like a ball, e.g., in order to connect with a support via a ball and socket joint configuration.

### FIFTH EMBODIMENT

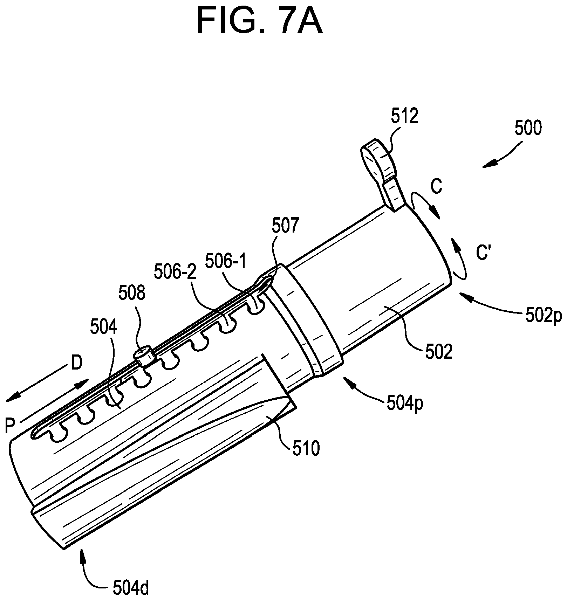

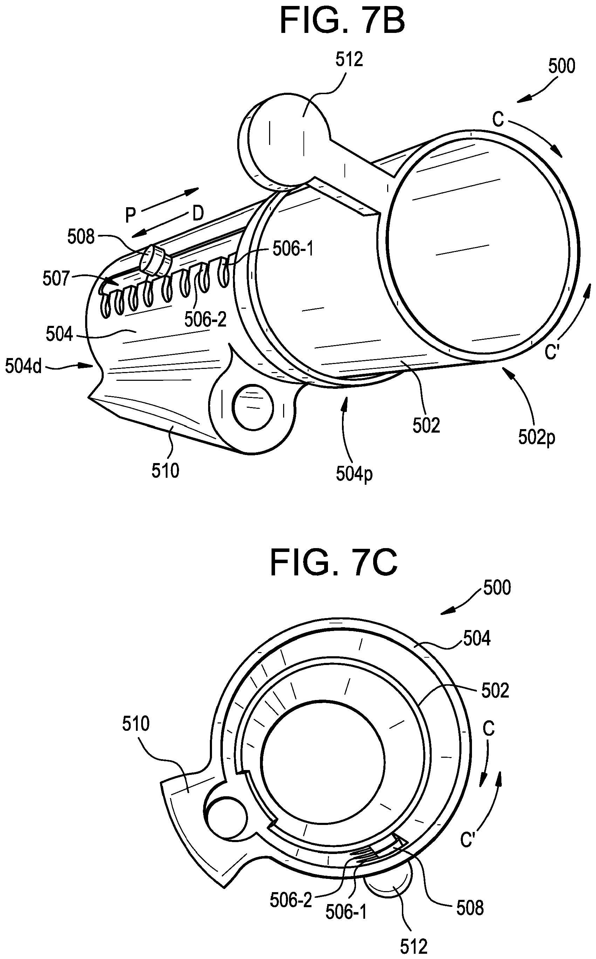

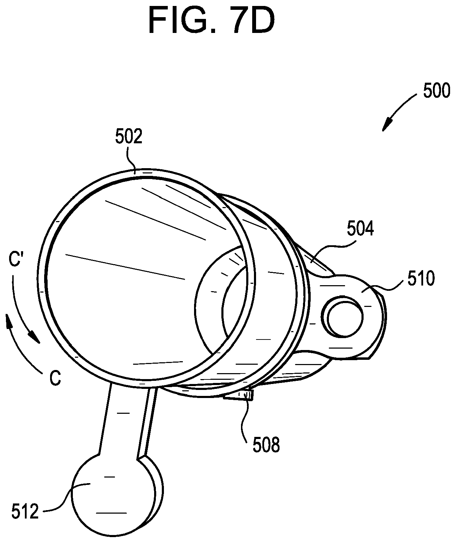

FIGs. 7A to 7D illustrate another exemplary embodiment of a surgical access device 500. The surgical access device 500 can include an inner tube 502 and an outer tube 504. The inner tube 502 and the outer tube 504 can include openings or working channels formed to and from their respective proximal and distal ends. The openings of the inner and outer tubes 502 and 504, defined by their respective inner surfaces, can vary in size and shape, as described above. The size and shape of the inner and outer tubes 502 and 504, as defined by their external surfaces, can likewise vary in size and shape, as described above. The inner and outer tube 502 and 504 can have similar shapes, and can be sized such that the internal surface of the outer tube 504 has a circumference slightly larger than the circumference of the external surface of the inner tube 502, such that the inner tube 502 can slide within the opening of the outer tube 504 without allowing any or a substantial amount of movement of the inner tube 502 other than along distal and proximal directions.

As shown in FIGs. 7A to 7D, the access device 500 can includes a turn-advance-turn-lock mechanism having a plurality of slots 506-1, 506-2, etc. (collectively "506") formed on the outer tube 504 and a knob 508 or a similar protrusion formed on the inner tube 502. In other arrangements, the slots 506 can be formed in the inner tube 502 and the knob 508 can be formed on the outer tube 504. The knob 508 can be a feature protruding from an external surface of the inner tube 502. The knob 508 can have a shape and size that matches the shape and size of the slots 506, such that the knob 508 can be slid into one of the slots 506 to prevent or resist relative distal and proximal movement between the inner and outer tubes. The thickness of the knob (e.g., the distance which the knob protrudes from the external surface of the inner tube 502) can be at least equal to the thickness of the body of the outer tube 504, as measured from its external surface to its internal surface. While the knob 508 can be provided in any position on the tube, in some embodiments, the knob 508 can be provided on or proximate to the distal end of the inner tube 502, enabling the length of the access device 500 to be maximized.

The access device 500 can include a handle 512 for controlling the sliding and rotation of the inner tube 502 in the manner described in further detail below, and/or for securing the access device 500 to a support in the manner described herein.

The slots 506 of the access device 500 can be formed as holes or perforations through the external and internal surfaces of the outer tube 504. The slots 506 can be formed at regular or irregular intervals along the length of the outer tube 504. The slots 506 can be disposed in the same line as the proximal and distal length of the outer tube 504. The slots 506 can be connected by a path 507, which can enable movement of the knob 508 between the slots 506. Although eight slots 506 are shown in FIGs. 7A to 7D, the outer tube 504 can include any number of slots. The path 507 can be an elongate slot having a longitudinal axis that is parallel or substantially parallel to the longitudinal axis of the access device 500. The slots 506 can be defined as lateral or circumferential extensions of the path 507, and can extend perpendicular to or obliquely from the path 507.

The outer tube 504 can include a sub-tube 510, which can be a tube that is formed on the external surface of the outer tube 504. The sub-tube 510 can be of a smaller size (e.g., length, circumference, and/or other dimensions) and/or shape as the outer tube 504. The size and shape of the sub-tube 510 can change from its distal end 510d to its proximal end 510p. An opening or working channel can be formed through the sub-tube 510, to and from its proximal and distal ends 510p and 510d. The opening can be configured to provide supplemental access to the patient's surgical area. For instance, the supplemental access provided by the opening can be used to insert an object or tool different than the object or tool inserted through the opening of the outer tube 504. Such separation of openings can be used when it is optimal to maintain the tools or objects separate from one another during surgery. In some embodiments, a camera system can be inserted through the sub-tube 510.

To slidably engage the inner and outer tubes 502 and 504, the inner tube 502 can be inserted into the outer tube 504. The inner and outer tubes 502 and 504 can be manufactured or produced such that the inner tube 502 is pre-inserted into the outer tube 504. To adjust the access device 500 to a desired length, the inner and outer tubes 502 and 504 can be moved or positioned relative to each other, and the desired length locked or fixed by inserting the knob 508 into one of the slots 506.

More specifically, to set the length of the access device 500, the inner tube 502 can be rotated about its central longitudinal axis relative to the outer tube 504 (in a clockwise direction C when viewed from a proximal vantage point) until the knob 508 is positioned on the path 507 and/or adjacent to the edge of the path 507 that is opposite the slots 506. With the knob 508 positioned on the path 507, the inner tube 502 can be moved distally or proximally, relative to the outer tube 504, along illustrated directions D and P. When the inner and outer tubes 502 and 504 are positioned such that the access device 500 is of a desired length, the inner tube 502 can be rotated about its central longitudinal axis relative to the outer tube 504 (in a counterclockwise direction C' when viewed from a proximal vantage point). Rotation of the inner tube 502 in a counterclockwise direction can cause the knob 508 of the inner tube 502 to slide from the path 507 into an adjacent one of the slots 506. If a desired length of the access device 500 results in the knob 508 being adjacent to a wall separating two of the slots 506, the inner tube 506 can be further moved distally or proximally to slide the knob 508 into one of the those two slots 506 (e.g., the slot closest to the knob 508). The inner tube 502 can be moved and rotated along directions C, C', P, and D using the handle 512.

With the knob 508 positioned in one of the slots 506, the distal end of the access device 500 can be inserted into a patient's body to reach a desired surgical region, without causing the length of the access device 500 to be modified. That is, the shape of the slots 506 can prevent the distal, proximal, and counterclockwise movement of the inner tube 502 and/or its knob 508 relative to the outer tube 504. Prior to or while the access device 500 is inserted in the patient's body, the length of the access device 500 can be adjusted in the same fashion, by rotating (e.g., clockwise) the inner tube 502, sliding it proximally or distally to achieve a desired length, and rotating the inner tube in the opposite direction (e.g., counterclockwise) to lock its position in another one of the slots 506. The illustrated access device 500 is configured such that the outer tube 504 is generally distal to the inner tube 502. In other arrangements, the inner tube 502 can be generally distal to the outer tube 504.

### SIXTH EMBODIMENT

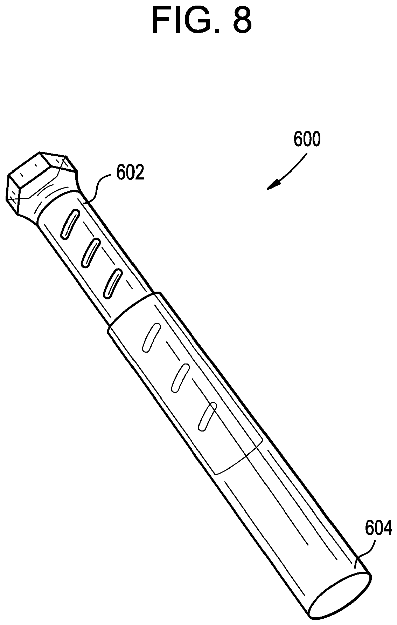

FIG. 8 illustrates another exemplary embodiment of a surgical access device 600. The surgical access device 600 can include an inner tube 602 and an outer tube 604 that are telescopically mated to one another and that define a working channel therethrough. The inner and outer tubes 602, 604 can include engagement features configured to maintain a relative longitudinal position of the inner and outer tubes. The engagements features can be threads that allow an overall length of the access device 600 to be adjusted by threading the inner tube 602 into or out of the outer tube 604. The engagement features can be configured to slip or skip when sufficient force is applied thereto. Accordingly, gross length adjustment can be achieved by simply pushing or pulling the inner and outer tubes with sufficient force to overcome the engagement features. Fine length adjustment can be achieved by threading the tubes into or out of one another..

An external surface of the inner tube 602 and an internal surface of the outer tube 604 can include complementary threads or similar engagement features. The threads can spiral around all or a part of the circumference of the external surface of the inner tube 602 and the internal surface of the outer tube 604. For example, the threads can be formed as an interrupted spiral thread, as shown, having a plurality of gaps between thread segments. The threads can be complementary to each other, such that a male or external thread on one of the tubes 602 or 604 engages with a female or internal thread on the other of the tubes 602 or 604. The threads can be provided on any amount of length on the tubes 602 and 604.

The length of the surgical access device 600 can be adjusted in one or more ways. For example, one way to adjust the length of the surgical access device 600 can be by pulling or pushing, in opposite proximal and distal directions, the inner tube 602 and the outer tube 604, with sufficient force to cause the thread of one of the tubes 602 or 604 to skip or slip over the thread of the other of the tubes 602 or 604. The pulling or pushing can be repeated in order to skip or slip as many threads as needed to place the inner and outer tubes 602 and 604 in a position relative to one another that results in the desired length of the access device 600. One or both tubes 602, 604, or the engagement features thereof, can be formed from a flexible or deformable material to facilitate such slipping. The dimensions or material properties of the tubes 602 and 604, or the engagement features thereof, can be selected to ensure that threads are not slipped or skipped when minimal or incidental force or pressure is applied on the tubes 602 and 604.

Another way to adjust the length of the surgical device can be to rotate the inner tube 602 clockwise or counterclockwise relative to the outer tube 604, e.g., about a central longitudinal axis of the access device 600. Rotating the inner tube 602 relative to the outer tube 604 can cause the threads of the inner and outer tubes 602 and 604 to advance into or out from one another to adjust an overall length of the access device 600. This manner of adjusting the length of the access device 600 can be more controlled and/or can allow for more precise small incremental length adjustment as compared with the above described thread skipping or sliding. The tubes 602 and 604 can be mated in a slight interference fit to resist or prevent relative rotation of the inner and outer tubes 602 and 604 when minimal or incidental rotational force is applied thereto.

When the access device 600 has been adjusted to have a desired length, the access device can be inserted into a patient's body to access a surgical region. The length of the access device 600 can also be adjusted while the access device is inserted into a patient.

### SEVENTH EMBODIMENT

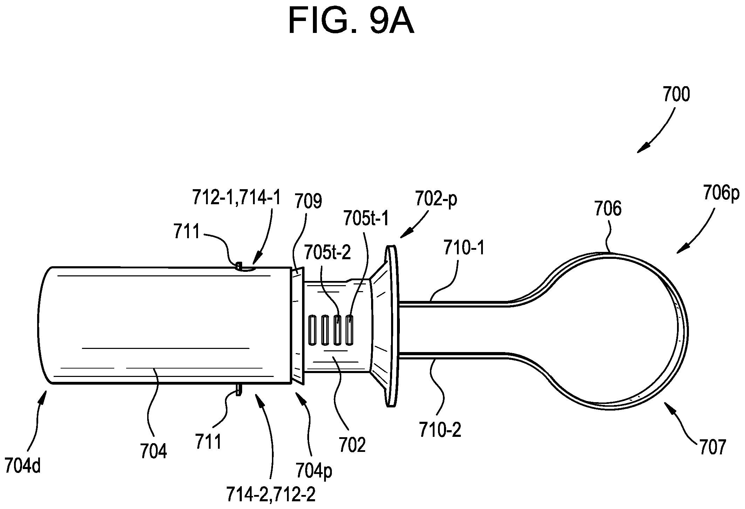

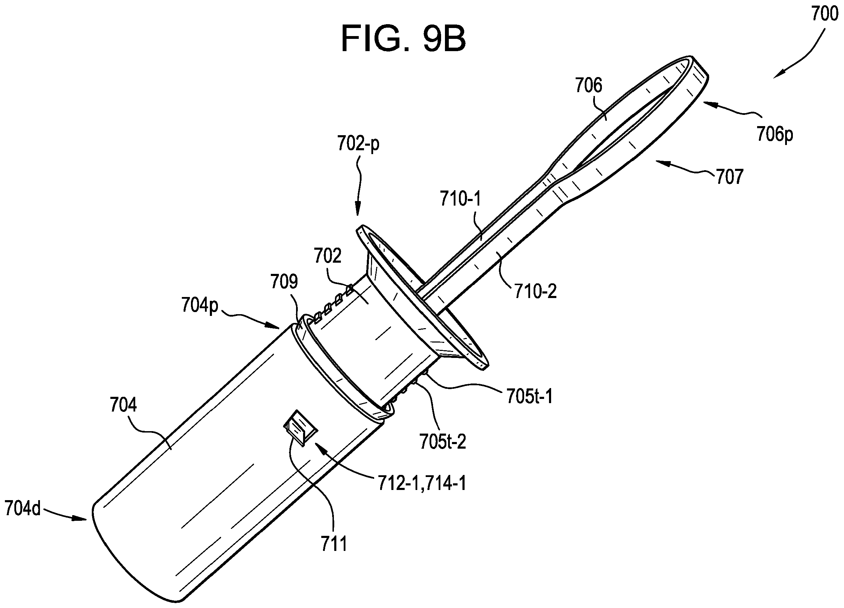

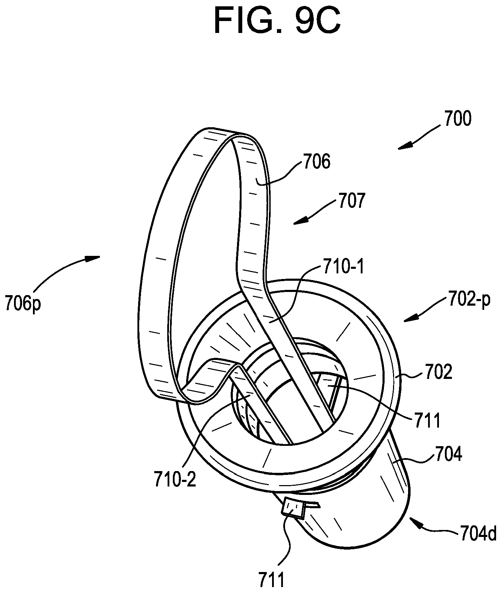

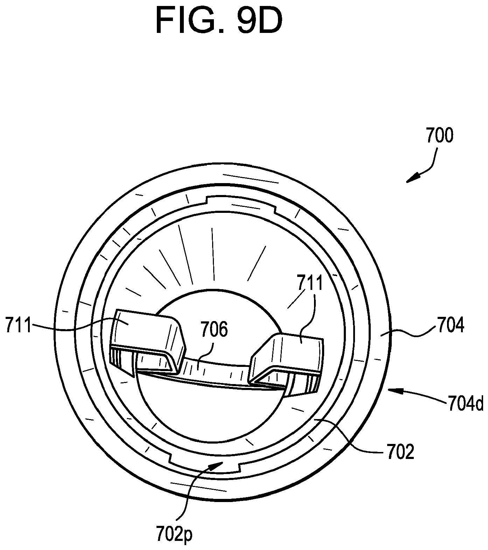

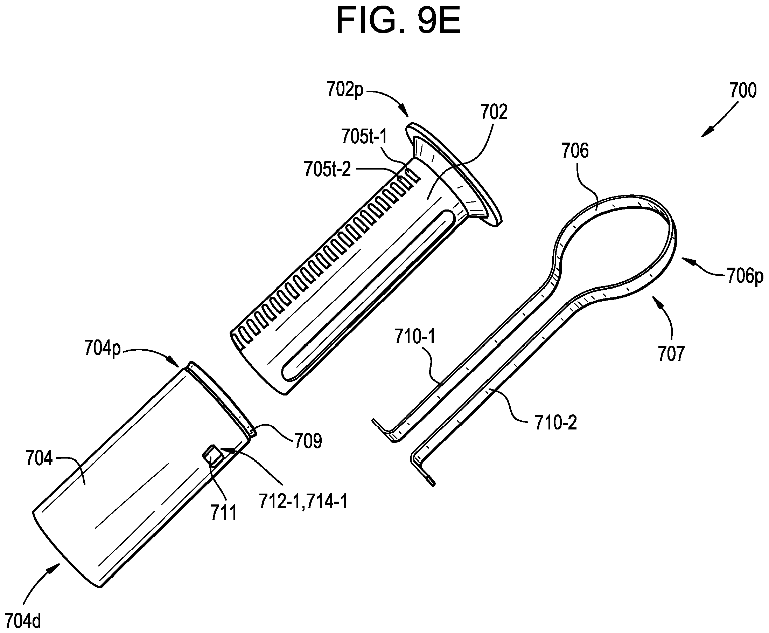

FIGs. 9A to 9E illustrate another exemplary embodiment of a surgical access device 700. The access device 700 can include an inner tube 702, an outer tube 704, and an auxiliary tool 706. As described in further detail below, the length of the surgical access device 700 can be adjusted by moving the inner tube 702 proximally and distally relative to the outer tube 704, and locking the position of the inner tube 702 relative to the outer tube using the auxiliary tool 706.

As shown, the inner tube 702 and the outer tube 704 can include openings or working channels formed therethrough, to and from their distal and proximal ends 702d and 704d, and 702p and 704p, respectively. The circumference of the opening of the outer tube 704 at the proximal end 704p can be larger than the circumference of an external wall of the inner tube 702 at the proximal end 702p, such that the inner tube 702 can be disposed within the outer tube 704 to form a telescoping, slidable engagement therebetween.

The proximal end 702p of the inner tube 702 can include or can be formed into a cone-shaped portion or flange. The flange can serve as a stop portion to prevent the distal movement of the inner tube 702, relative to the outer tube 704, beyond the inner tube's proximal end 702p. Such a flanging structure of the proximal end 702p of the inner tube 702 can also allow the proximal end of the inner tube 702 to be positioned tightly on the surface of the skin of a patient when the access device 700 is inserted into the patient's body.

The inner tube 702 can include one or more ratchet features 705 disposed or formed on the length of the external surface of the inner tube 702. The ratchet features 705 can be disposed on diametrically opposed sides of the external surface of the inner tube 702. The ratchet features 705 can include a plurality of tooth features (705t-1, 705t-2, etc.; collectively referred to as "705-t"), which can define male engagement features protruding from the external surface of the inner tube 702.

The tooth features 705t of the ratchet 705 can be configured to engage with or be received within a lip, groove, gap or other like structure 709 formed in the internal surface of the outer tube 704, as described in further detail below. The tooth features 705t can be formed along part or the entire length of the inner tube 702. The tooth features 705t can be formed along part of the external surface of the inner tube 702 starting at a lengthwise position proximate to the proximal end 702p of the inner tube 702. The size and shape of the tooth features 705t can vary. The distance or spacing between tooth features 705t can be the same or vary between each of the tooth features 705t. The shape (e.g., curve, square, triangle), thickness (e.g., amount of protrusion away from the external surface), spacing and/or height (e.g., distance spanned across the length of the external surface of the inner tube 702 by each tooth) of the tooth features 705t can depend on the shape, thickness, distance and/or height of the lip 709 formed on the internal surface of the outer tube 704 and vice versa. The width of the tooth features 705t can be the same or vary between each of the tooth features, and they can spread over part or the entire circumference of the external surface of the inner tube 702.

The outer tube 704 can include an annular groove or lip 709 for engaging with the ratchet 705 and/or its tooth features 705t. As shown in FIGs. 9A to 9D, the lip 709 can be provided around part or the entire circumference of the internal surface of the outer tube 704. The lip 709 can be formed at or proximate to the proximal end 704p of the outer tube 704, such that, when engaged with the inner tube 702, the resulting length of the access device 700 can benefit from the rest of the length of the outer tube (e.g., from the lip 709 at the proximal end 704p to the distal end 704d).

The lip 709 can engage with the tooth features 705t to limit or prevent distal and proximal movement of the inner tube and the outer tube 702 and 704 relative to each other. For example, the lip 709 can be a concave feature such as a groove or gap formed on the internal surface of the outer tube 704, extending from the internal surface toward the external surface of the outer tube 704 such that the groove or gap is of sufficient depth to accommodate part or all of a tooth feature 705t. In another example, the lip 709 can be formed by the shape of the proximal end 704p of the outer tube 704. For instance, when the proximal end 704p of the outer tube 704 has a cone shape, the internal surface of the outer tube 704 where the cone shape ends and meets the rest of the internal surface of the outer tube can be convex and/or have a smaller circumference than the rest of the outer tube 704. This narrower or convex portion of the internal surface of the outer tube 704 can be configured to have a circumference smaller than the circumference of the outer-most portion of the tooth features 705t and larger than the circumference of the external surface of the inner tube 702 at the regions between the tooth features 705t. Such a configuration can allow the narrower portion of the internal surface of the outer tube 704 to be received between two of the tooth features 705t, fixing the position of the outer tube 704 and limiting or preventing the distal and proximal movement of the inner and outer tubes 702 and 704 relative to one another when minimal or incidental force is applied in a proximal or distal direction to the inner and/or outer tubes 702, 704.

To adjust the length of the access device 700 using the ratchet mechanism 705, force can be applied to the inner tube 702 and the outer tube 704 in opposite distal or proximal directions. In other words, pulling the inner tube 702 away from the outer tube 704, pulling the inner tube 702 proximally and the outer tube 704 distally, or pushing the inner tube 704 distally toward the outer tube 704, with sufficient force, can cause the lip 709 on the internal surface of the outer tube 704 to skip or slip past one or more of the tooth features 705t. The pulling and/or pushing of the tubes 702 and/or 704 can be repeated until the lip is fixedly engaged with one or more of the tooth features 705t at a position where the access device 700 is at a desired length. The amount of force needed to slip or skip the lip 709 past a tooth feature 705t can depend on various factors, including the size and/or shape of the lip 709 and/or tooth features 705t, and/or the material of the inner and outer tubes 702 and 704.

An auxiliary tool 706 can be included in the access device 700 to facilitate extension or retraction of the tubes 702, 704 relative to one another. The auxiliary tool 706 can be provided as a length-adjustment feature in addition or alternative to the ratchet 705. As shown in FIGs. 9A to 9D, the auxiliary tool 706 can include a handle portion 707 on the proximal end 706p of the auxiliary tool 706. The auxiliary tool 706 can include two prongs 710 (710-1, 710-2) that extend distally from the handle 707. The distal end of the prongs 710 can include or can be formed as tabs 711 that curve or bend away from one another in a radially-outward direction. The length of the tabs (e.g., the distance that the tabs 711 extend away from their respective prongs 710) and other of their dimensions (e.g., width, thickness) can be based on the thickness of the inner and outer tubes 702 and 704, and/or the size of respective slots 712 and 714 formed on the inner and outer tubes 702 and 704. For example, the tabs 711 can be configured such that they can penetrate through the slots 712 and 714.

The auxiliary tool 706 can be designed such that it is self-spreading, meaning that the prongs 710 are biased apart, e.g., by forming the auxiliary tool from a flexible and resilient material. In a configuration where the prongs 710 are biased apart, the distal ends of the tabs 711 can be separated, in their resting state, by a distance that is larger than the diameter of the internal and external surfaces of the inner and outer tubes 702 and 704. The prongs 710 can be squeezed or compressed, against their bias, to narrow the distance separating the prongs 710 and/or the distal ends of the tabs 711. When squeezed, the prongs 710 and tabs 711 can be separated by a distance smaller than the diameter of the inner tube 702, such that the prongs 710 and 711 can be moved through the inner tube 702 (and/or the outer tube 704), to be inserted through the slots 712 and 714.

The inner tube 702 can include one or more slots 712 (712-1, 712-2) that penetrate through the external surface and internal surface of the inner tube 702. The number of slots 712 can correspond to the number of prongs 710 or tabs 711 provided on the auxiliary tool 706. The slots 712 can be provided on diametrically opposed sides of the inner tube 702, or otherwise to align with the tabs 711 - e.g., so that when one of the tabs 711 is positioned through one of the slots 712, another of the tabs 711 can also extend through another of the slots 712 without manipulating the shape of the auxiliary tool 706. The slots 712 can be provided along part of the length of the inner tube 702. The slots 712 can extend from a region proximate to the proximal end 702p toward the distal end 702d. The width of the slots 712 can be at least as large as the width of the tabs 711.

The outer tube 704 can include one or more slots 714 (714-1, 714-2) that penetrate through the external surface and internal surface of the outer tube 704. The number of slots 714 can correspond to the number of prongs 710 or tabs 711 provided on the auxiliary tool 706. The slots 714 can be provided on diametrically opposed sides of the outer tube 704, or otherwise to align with the tabs 711 - e.g., so that when one of the tabs 711 is positioned through one of the slots 714, another of the tabs 711 can also extend through another of the slots 714 without manipulating the shape of the auxiliary tool 706. The width of the slots 714 can be the same as or similar to that of the slots 712, meaning at least as large as the width of the tabs 711. The length of the slots 714 (e.g., the distance measured in a proximal to distal direction) can be smaller than the length of the slots 712. The length of the slots 714 can be equal to or slightly larger than the thickness of the tabs 711 (e.g., 200%, 300%, 500%). While the slots 714 can be disposed anywhere along the length of the outer tube 704, in some embodiments, the slots 714 can be formed proximate to the proximal end 704p of the outer tube 704, such that the length of the access device 700 can be optimized.

To adjust the length of the access device 700 using the auxiliary tool 706, the distal end 702d of the inner tube 702 can be inserted within the opening at the proximal end 704p of the outer tube 704. The auxiliary tool 706 can be inserted within the inner tube 702. The slots 712 and 714 of the inner and outer tubes 702 and 704 can be aligned with one another, such that the openings formed by the slots 712 and 714 extend through the inner and outer tubes 702 and 704. Because the prongs and tabs 710 and 711 can be biased apart to a distance that exceeds the diameter of the opening of the inner tube, the prongs of the auxiliary tube 706 can be squeezed or compressed toward one another in order to insert the auxiliary tool 706 into the opening of the inner tube 702. The prongs 710 can then released from their squeezed state, causing the prongs 710 and tabs 711 to spread apart, such that the tabs 711 extend or penetrate through the slots 712 and 714.

With the tabs 711 extending through the slots 712 and 714, the auxiliary tool 706 can be pulled or pushed proximally and distally, using the handle 707, to adjust the length of the access device 700. Because the slots 714 are shorter than the slots 712, pulling or pushing the auxiliary tool 706 causes the proximal or distal surfaces, respectively, of the tabs 711 to engage with the proximal or distal surfaces of the slots 714 of the outer tube 706. Applying a pulling or pushing force on the proximal or distal surfaces of the slots 714 using the tabs 711 can cause the outer tube 704 to move proximally or distally, respectively, relative to the inner tube 702 along the length of the slots 712. When the inner and outer tubes 702 and 704 are positioned relative to one another such that a desired length of the access device 700 is achieved, the pulling or pushing of the auxiliary tool can be ceased. The auxiliary tool 706 can thus be used to remotely or indirectly apply a distally-directing pushing force or a proximally-directing pulling force to the outer tube 704 to move the outer tube axially relative to the inner tube 702. This can be particularly useful when adjusting the length of the access device 700 while it is inserted into a patient, when the outer tube 704 may be disposed deep within the patient, may be surrounded with tissue, or may otherwise be in a position that makes it difficult to manipulate directly. In use, the access device 700 can be adjusted to a first length and can be inserted through an incision formed in the skin of the patient and advanced distally until the flange on the proximal end of the inner tube 702 abuts the skin surface. The auxiliary tool 706 can then be used to apply a distally-directed force to the outer tube 704 to extend the length of the access device 700 to a second length that is greater than the first length. For example, the outer tube 704 can be advanced distally until it bottoms out against a bone surface or other target location. The auxiliary tool 706 can be removed from the access device 700 to provide clear access through the working channel of the access device, or can remain in place during the surgery.

In embodiments in which the access device 700 is also equipped with one or more ratchet features 705, the ratchet features can limit or prevent distal and proximal movement of the inner and outer tubes 702 and 704 relative to one another. In another arrangement, the tabs of the auxiliary tool can engage ratchet teeth on the outer tube. Pulling the tabs radially-inward can disengage them from the ratchet teeth, allowing for length adjustment. Releasing the tabs can allow them spring into engagement with the teeth to limit or prevent length adjustment.

### EIGHTH EMBODIMENT

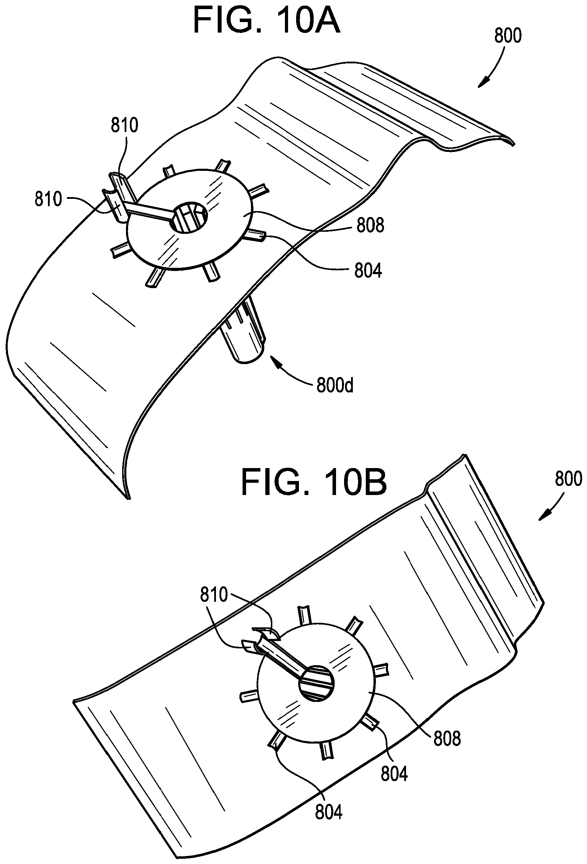

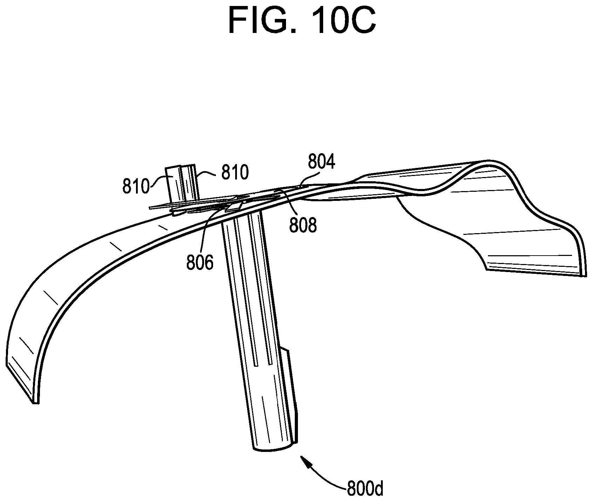

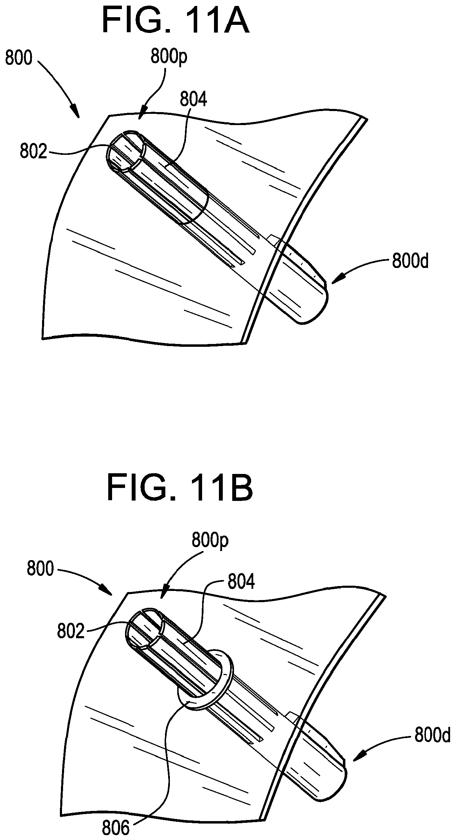

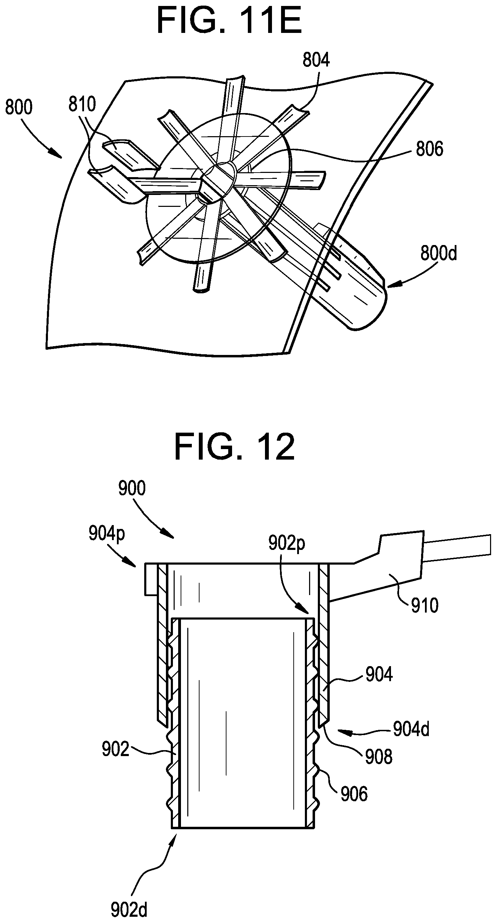

FIGs. 10A to 10C, and 11A to 11E illustrate another exemplary embodiment of a surgical access device 800. The access device 800 can include a tube or main body having an opening or working channel formed from a proximal end 800p to a distal end 800d. Slits 802 can be formed longitudinally through the body of the access device 800. The slits can penetrate through the external and internal surfaces of the access device 800. The slits 802 can extend from the proximal end 800p distally along the length of the access device 800 to an area prior to the distal end 800d. Bendable arms 804 can be formed longitudinally by the body of the access device 800 positioned between two of the slits 802. In other words, the bendable arms 804 can extend longitudinally along the length of the access device 800, from the proximal end 800p and parallel to the slits 802. While the width of the slits 802 and the bendable arms 804 can vary, the width of the bendable arms 804 can be larger than the width of the slits 802. The bendable arms 804 can allow the length of the access device 800 to be adjusted to a desired or optimal length to reach or provide access to a surgical area in the patient's body, as explained in further detail below.

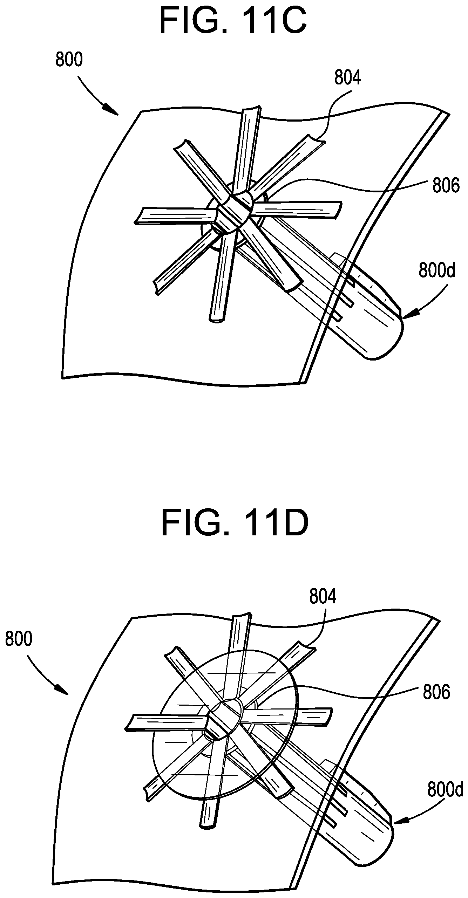

To operate the access device 800 and adjust its length, the distal end 800d of the access device can be inserted into the patient's body to a depth sufficient or optimal to access the surgical area in the patient's body, as shown in FIG. 11A. When the access device 800 is so positioned, the bendable arms 804 extending from the proximal end 800p of the access device 800 can be bent at or relative to a pivot point at or proximal to the surface of the patient's skin, as shown in FIG. 11C.

The access device 800 can include a support ring 806. The support ring 806 can be slidably disposed around the external surface of the access device 800, e.g., with sufficient friction such that the ring 806 cannot be proximally or distally moved relative to the access device 800 without applying force thereto. To facilitate the bending of the bendable arms 804, the ring can be inserted over the access device 800 and slid along an exterior surface thereof to position the ring in contact with the skin surface, as shown in FIG. 11B, or at any other desired longitudinal position along the access device. The ring 806 can be positioned such that its distal end is below the surface of the patient's skin, and its proximal end is above the surface of the patient's skin. The ring 806 can be positioned entirely beneath the skin, or can be spaced proximally from the skin. The bendable arms 804 can be folded over the ring, allowing the arms to be more easily and evenly bent against the edge of the proximal side of the ring 806, as shown in FIG. 11C. With the arms 804 bent at or proximate to the surface of the patient's skin, distal movement of the access device 800 relative to the patient's body can be prevented.

To stabilize the access device 800 and prevent its movement once the bendable arms 804 have been bent, one or more of the arms can be attached or affixed to the patient, e.g., to the skin of the patient. For example, an adhesive, tape, sutures, or other fixing tools or techniques can be applied over one or more of the bendable arms 800, as shown in FIG. 11D. In some embodiments, as shown in FIG. 10A, a circular adhesive disc 808 can be placed over the bendable arms 800, such that an adhesive side of the disc 808 contacts the bendable arms 800 and the surface of the patient's skin, thereby fixing them to one another. The adhesive 808 can prevent distal or proximal movement, or rotation, of the access device 800 relative to the patient. The adhesive disc 808 can include a central opening to allow access to the working channel of the access device 800.

Additionally or alternatively, the access device 800 can be stabilized to the patient, for example by securing the access device 800 to an anatomical structure of the patient or an implant implanted in the patient. The anchor point can be remote from the access device. The anchor point can be a bone anchor, such as a pedicle or lateral mass screw. The anchor point can be an extension tab or post 810 extending proximally from a bone anchor implanted in the patient to a position outside the patient. The anchor point can be on an ipsilateral or contralateral side of the spine from the access device 800. The anchor point can be a post 810 fixed to a patient's pedicle or other bone and that penetrates through the patient's skin at a region sufficiently close to the access device 800 that the post can receive one or more of the bendable arms 804. The post 810 can be positioned such that a bendable arm 804 can be folded over and positioned between opposed arms of the post, as shown in FIG. 10A. Securing the access device 800 can preventing its radial movement or rotation, and otherwise maintain the access device at a fixed position and/or orientation with respect to the patient. The opposed arms of the post 810 can be spaced apart from one another a distance that is larger than the width of the bendable arm 804, such that the bendable arm 804 can be positioned in the space between the arms. A set screw, fastener, or other closure mechanism can be applied to the post 810 to secure the bendable arm therein. With the bendable arms 804 stabilized, the access device 800 can be operated with minimal or no movement or rotation relative to the patient's body.

### NINTH EMBODIMENT

FIG. 12 illustrates another exemplary embodiment of a surgical access device 900. The surgical access device 900 can include an inner tube 902 and an outer tube 904. The inner tube can include proximal and distal ends 902p and 902d; and the outer tube can include proximal and distal ends 904p and 904d. The inner tube 902 and the outer tube 904 can have respective holes or working channels formed therethrough, from their distal to their proximal ends. The inner tube 902 can be slidably engaged within the outer tube 904, such that the two are telescopically mated.

The external surface of the inner tube 902 can include ribs or other engagement features 906 wrapping around all or a part of the circumference of the external surface of the inner tube 902. The internal surface of the outer tube 904 can include counterpart ribs or other engagement features 908 that wrap around all or a part of the circumference of the internal surface of the outer tube 904. The ribbed features 906 and 908 can be convex or concave structures, and the ribbed features of the inner tube 902 can engage with the ribbed features of the outer tube 902. For example, convex ribbed features can engage with two opposing convex features (e.g., by being positioned between the two convex features), or can engage with an opposing and complementary concave feature (e.g., by being positioned within the concave feature).

The shape and size of the ribbed features 906 or 908 can be based on the shape and size of the opposite and complementary ribbed features 906 and 908. For example, convex features can protrude, away from the external surface of the inner or outer tube 902 and 904, a distance equal to the depth of a complementary concave feature, or a distance equal to the amount of protrusion of the complementary convex features. The shape, size, and/or material properties of the ribbed features can be selected to allow the ribbed features 906 and 908 to skip or slip past or over each other as the inner and outer tubes 902 and 904 are moved proximally and distally relative to one another when opposite distal and proximal forces are applied to the inner tube 902 and the outer tube 904. In other words, the ribbed features 906 and 908 can function like a ratchet by allowing the ribbed features to skip or slip one another when proximal and/or distal force is applied to the inner and outer tubes 902 and 904.

The proximal end 904p of the outer tube can include attached thereto or formed thereon a collar or handle 910 portion that can enable the access device 900 to be connected to an anchor or similar stabilization device. The anchor or stabilization device can have a fixed position relative to on object or the patient's body.

The length of the access device 900 can be adjusted prior to or during the access device 900 being inserted into the patient's body.

### TENTH EMBODIMENT

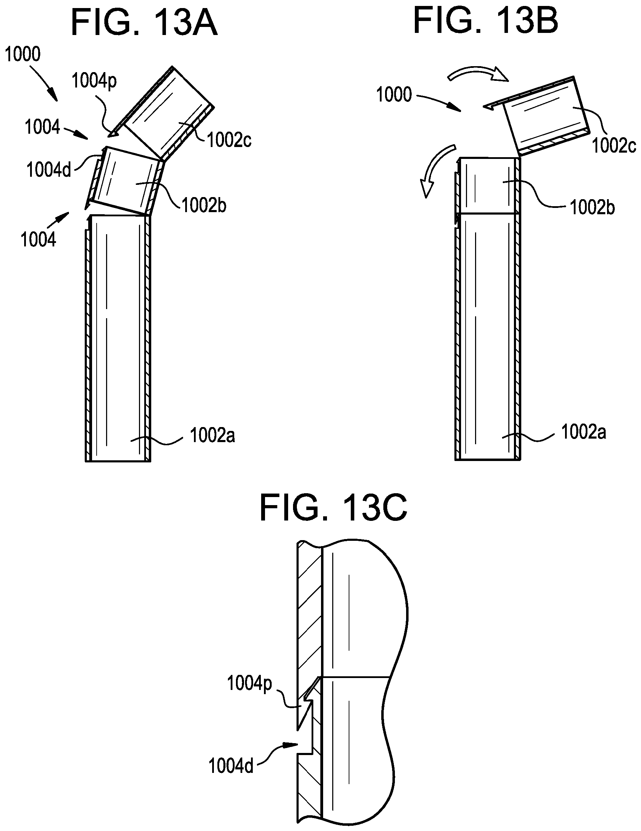

FIGs. 13A to 13C illustrate another exemplary embodiment of a surgical access device 1000. The surgical access device 1000 can include a tube having a distal end and a proximal end 1000d and 1000p, respectively. A part of the tube can be formed from or can include a plurality of segments, such as segments 1002a, 1002b and 1002c (collectively "1002"). As explained below, the segments 1002 can be attached and/or detached from one another to achieve a tube of a desired or optimal length for surgery. The segment at the distal end 1000d - namely segment 1002a in FIG. 13A, can be the longest of the segments and the one that is to be inserted into the patient's body for surgery.

The segments 1002 can be attachable and detachable using film joint and/or snap fit features 1004 that connect two of the segments 1002. The snap fit features 1004 can include a protruding part 1004p (e.g., hook, stud, bead) on one segment and a mating depression part (e.g., undercut) 1004d on the other segment. The protruding part and depression parts can be provided on either a distal or a proximal one of two segments. Two segments can be connected by one or more snap fit features 1004. The length of the protruding part can be configured to be long enough to reach the corresponding depression. The depth of the depression can be large enough to accommodate the protruding part.

Two segments can be detached by pulling the two segments apart from each other. The snap fit features 1004 can be configured such that a predetermined amount of force allows the two segments to be separated without extreme or machine force being needed. The force to be applied to separate two segments can be a pulling force away from the two parts of the snap features 1004, while pivoting at a diametrically opposed end of the two segments. Two segments can be attached by pushing the two segments toward each other, such that the two parts of the snap fit features 1004 can snap within one another.

When two segments are connected, the snap fit features can be configured to create or maintain a smooth, constant external surface of the access device 1000. Segments can be detached or attached as described above until a desired length of the access tube 1000 for surgery is achieved. The segments can be formed from a flexible or bendable material, such that the snap fit features are deformable during assembly or disassembly to allow the segments to be attached or detached. The segments can be formed from a rigid material and can be permanently broken when separated from one another.

### ELEVENTH EMBODIMENT

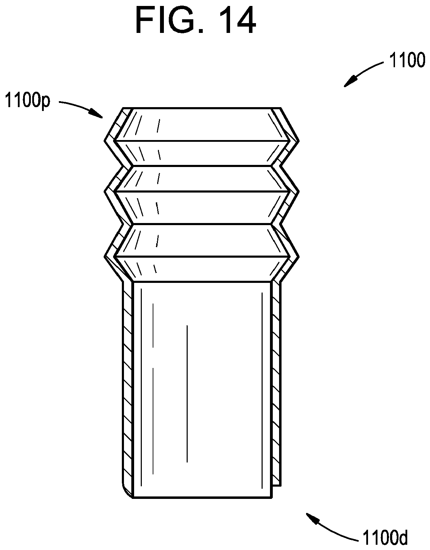

FIG. 14 illustrates another exemplary embodiment of a surgical access device 1100. The surgical access device 1100 can include a tube having a distal end 1 100d and a proximal end 1100p. Part of the access device 1100 proximate to the proximal end 1100p can be circumferentially corrugated with bellows or accordion features 1102 similar to those of a drinking straw.

The length of the access device 1100 that is corrugated can vary as needed to achieve a desired stability and length of the access device 1000. Likewise, the inward depth and/or protrusion of the corrugations can vary in order to achieve a desired strength of each corrugated feature, and/or to adjust the force needed to shorten and lengthen the access device 1000.

The access device 1000 can be lengthened by applying axial tension or pulling force to opposite sides of the corrugated features. The access device 1000 can be shortened by applying axial compression or pushing force to opposite sides of the corrugated features. The pulling and pushing forces can be repeated until the desired length of the access device 1000 is achieved.

### TWELFTH EMBODIMENT

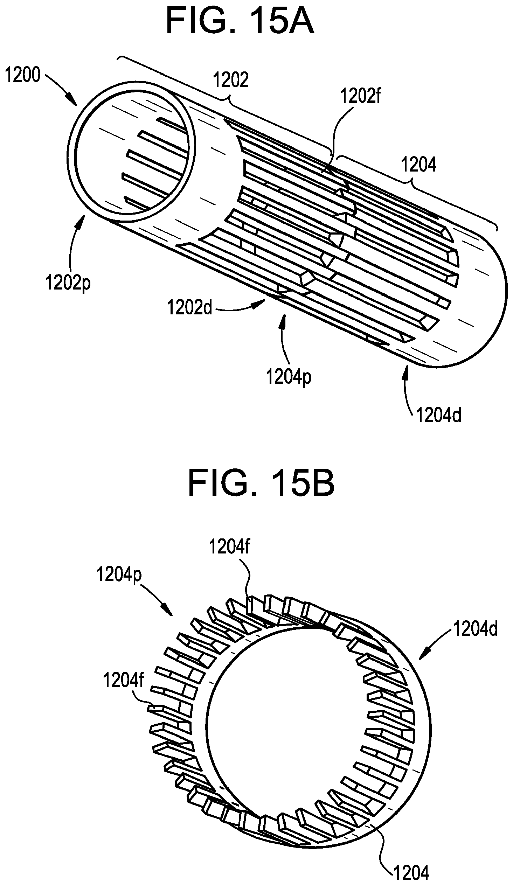

FIGs. 15A and 15B illustrate another exemplary embodiment of a surgical access device 1200. The access device 1200 can include two or more tubes 1202 and 1204. The tubes 1202 and 1204 can be connected via interdigitating fins or fingers 1202f and 1204f, respectively, as described below. The fingers can engage one another with sufficient friction to maintain the access device 1200 at a fixed overall length when subjected to forces typically associated with surgery through an access device. The length of the access device 1200 can be adjusted by sliding the proximal and distal tubes 1202, 1204 relative to one another to increase the engaged length of the fingers (and reduce the overall length of the access device) or to decrease the engaged length of the fingers (and increase the overall length of the access device).

As shown in FIGs. 15A and 15B, the tubes 1202 and 1204 can have respective distal ends (1202d, 1204d) and proximal ends (1204p, 1204p). One or more of the ends of the tubes 1202 and 1204 can include cantilevered fingers 1202f and 1204f that extend from the other end of the tube. For instance, in FIG. 15B, the tube 1204 is shown. The distal end 1204d can define a fully closed ring, from which the fingers 1204f extend toward and/or to the proximal end 1204p. The fingers 1204f can extend from the distal end 1204d or from an area between the distal end 1204d and the proximal end 1204p.

The fingers 1204f have a same size and shape as one another, and can have the same size and shape as the spaces or gaps separating them. Likewise, the size and shape of the fingers 1202f, and of the gaps separating the fingers 1202f, can match each other and the fingers 1204f. The fingers 1202f and 1204f can have a slightly different size than the gaps separating them, allowing the fingers of one of the tubes 1202 and 1204 to slide within or between the gaps of the other tube 1202 and 1204. The sizes and shapes can be configured such that a sufficient amount of surface area and associated friction between fingers of one tube and fingers of another tube can be achieved, to prevent the distal and proximal movement of the tubes 1202 and 1204 relative to one another when minimal or incidental force is applied thereto.

That is, the fingers and gaps of the tubes 1202 and 1204 can be designed such that they can engage with each other by sliding therebetween. In other words, the fingers 1202f of one tube 1202 can slide between the fingers 1204f of the other tube 1204. The two tubes can be pulled from and pushed toward one another to engage a shorter or larger length of the fingers 1202f and 1204f, until the desired length of the access device 1200 is achieved.

The inner diameters or circumferences of the tubes 1202 and 1204 can be the same. Thus, when the fingers 1202f and 1204f are engaged, the inner diameter or circumference of the access device 1200 can remain constant throughout its length, forming a continuous and smooth internal surface of the access device 1200. The outer diameters or circumferences of the tubes 1202 and 1204 can be the same. Thus, when the fingers 1202f and 1204f are engaged, the outer diameter or circumference of the access device 1200 can remain constant throughout its length, forming a continuous and smooth external surface of the access device 1200.

The access device 1200 can advantageously maximize the working channel inside diameter available for a given external diameter and wall thickness of the access device. In other words, because the proximal and distal tubes can have the same internal diameter and the same external diameter, space that would be otherwise occupied by one of the tubes in a telescoping-type access device is free to be used as part of the working channel.

### CONNECTION FEATURES

Any of the access devices described herein can include a connection feature. The connection feature can be used to secure the access device relative to the patient or another reference. The connection feature can be a spherical body connected to the access device by a cantilevered beam. The connection feature can be disposed adjacent a proximal end of the access device. The connection feature can be an external surface of the access device configured to be received between opposed jaws of a connector or clamp. The connection feature can be a recess or cut-out formed in a sidewall of the access device.

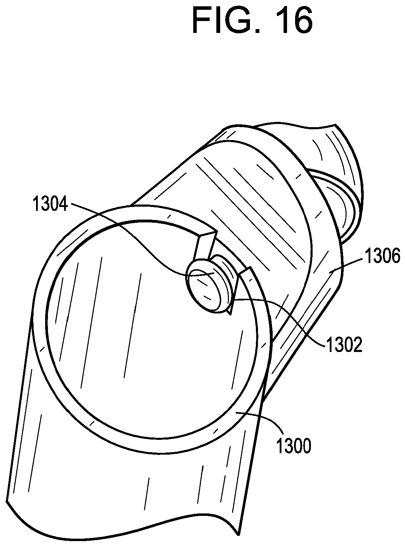

FIG. 16 illustrates an exemplary embodiment of a connection feature 1302 and counterpart connector. Any of the access devices described herein can include the connection feature 1302

The connection feature can be defined by a cutout 1302 formed in a sidewall of an access device 1300. The cutout 1302 can be open to a proximal end 1300p of the access device and can extend toward a distal end of the access device. The cutout 1302 can penetrate from an external surface of the tube 1300 to the internal surface. The cutout 1302 can include a path or slot portion extending from the proximal end 1300p, and a circular resting potion at the distal most end of the cutout. The distal portion of the cutout 1302 can have a chamfered or conical bearing surface.

The cutout 1302 can be configured to receive a counterpart mating feature of a connector or other support. The counterpart mating feature can include a cone 1304 protruding from a cap 1306. The cone 1304 can have a size and shape that enables the cone or a portion of the cone to slide within the cutout 1302. The cone 1304 can protrude from cap 1306 a length equal to the thickness of the body of the tube of the surgical access device 1300. The cone 1304 can be extendable and retractable from the cap 1306, to allow the cone to be extended when being inserted into the cutout 1302, and retracted to form a tight connection with the tube 1300. The cone 1304 can be coupled to a wire or cable, and tension can be applied to the cable to pull the cone 1304 towards the cap 1306 and into firm engagement with the bearing surface of the cutout 1302. The cone 1304 can taper at an angle that matches that of the chamfered portion of the cutout 1302. The cap 1306 can define a semi-cylindrical recess sized to receive at least a portion of the exterior surface of the access device 1300 therein. The connection feature can form a "zero-profile" connection, such that no portion of the connection feature protrudes from an internal or external surface of the access device 1300.

The cutout 1302 can be provided on one or more tubes of the access devices described herein. The cone and cap 1304 and 1306 can be adjusted to engage with multiple tubes simultaneously.

Any of the devices described herein can include access tubes that vary in structure or operation from what is shown. Any of the devices herein can include an access tube in the form of a closed tubular body. Any of the devices herein can include an access tube in the form of a multi-bladed retractor. The retractor can be configured to radially expand and/or contract. The retractor can include blades that can be toed inward or outward. Exemplary access devices that can be used with, or having features that can be included in, any of the devices herein are described in U.S. Patent No. 7,491,168, titled "Surgical Retractor Systems and Illuminated Cannulae," issued on February 17, 2009. Any of the devices herein can include an access tube that forms less than a full/closed circle, or that has a non-circular transverse cross-section.

The devices herein can be used to deliver flowable material to a site within a patient. The flowable material can be a hemostat. The flowable material can be a gelatin. The flowable material can be SURGIFLO hemostat matrix available from ETHICON. The devices herein can be used to deliver a powdered material to a site within a patient. The powdered material can be a powdered antibiotic, such as vancomycin. The powdered material can be powdered pig bladder. The powdered material can be so-called "pixie dust" or "pixie powder."

It should be noted that any ordering of method steps expressed or implied in the description above or in the accompanying drawings is not to be construed as limiting the disclosed methods to performing the steps in that order. Rather, the various steps of each of the methods disclosed herein can be performed in any of a variety of sequences. In addition, as the described methods are merely exemplary embodiments, various other methods that include additional steps or include fewer steps are also within the scope of the present disclosure.

The devices disclosed herein can be constructed from any of a variety of known materials. Exemplary materials include those which are suitable for use in surgical applications, including metals such as stainless steel, titanium, nickel, cobalt-chromium, or alloys and combinations thereof, polymers such as PEEK, ceramics, carbon fiber, and so forth. The various components of the devices disclosed herein can be rigid or flexible. One or more components or portions of the device can be formed from a radiopaque material to facilitate visualization under fluoroscopy and other imaging techniques, or from a radiolucent material so as not to interfere with visualization of other structures. Exemplary radiolucent materials include carbon fiber and high-strength polymers.

The devices and methods disclosed herein can be used in minimally-invasive surgery and/or open surgery. While the devices and methods disclosed herein are generally described in the context of spinal surgery on a human patient, it will be appreciated that the methods and devices disclosed herein can be used in any type of surgery on a human or animal subject, in non-surgical applications, on non-living objects, and so forth.

While various example embodiments have been described above, it should be understood that they have been presented by way of example, and not limitation. It is apparent to persons skilled in the relevant art(s) that various changes in form and detail can be made therein. Thus, the disclosure should not be limited by any of the above described example embodiments.

In addition, it should be understood that the figures are presented for example purposes only. The architecture of the example embodiments presented herein is sufficiently flexible and configurable, such that it may be utilized and navigated in ways other than that shown in the accompanying figures.

One skilled in the art will appreciate further features and advantages of the disclosure based on the above-described embodiments. Accordingly, the disclosure is not to be limited by what has been particularly shown and described. The scope of the invention is therefore defined by the appended claims.

## Claims

1. A surgical access device comprising:
an inner tube (702) including one or more engagement features of a first type disposed on an outer surface thereof and wherein the inner tube (702) comprises one or more slots (712) formed along part of the length of the inner tube, through the external and internal surfaces of the inner tube (702); and
an outer tube (704) telescopically mated with the inner tube (702), and including one or more engagement features of a second type formed at least on the inner surface thereof, wherein the outer tube comprises one or more slots (714) formed along part of the length of the outer tube, through the external and internal surface of the outer tube (704);
wherein the first type of engagement features and the second type of engagement features are complementary to each other, and
an auxiliary tool (706) comprising at least one tab (711) configured to engage with the at least one slot (714) formed in the outer tube (704) and the at least one slot (712) formed in the inner tube (702) to facilitate extension or retraction of the outer tube (704) relative to the inner tube (702);
wherein a length of the surgical access device is adjusted by applying opposite proximal or distal forces on the inner tube (702) and outer tube (704), thereby causing engagement features of the inner tube to slip from the corresponding one or more engagement features of the outer tube to adjacent others of the one or more engagement feature of the outer tube.

2. The surgical access device of claim 1, wherein the one or more engagement features of the inner tube (702) and the outer tube form oblique angles relative a longitudinal axis of the inner tube and/or the outer tube (704).

3. The surgical access device of claim 1 or claim 2, wherein the one or more engagement features of the inner tube (702) and the outer tube (704) are formed of a deformable material enabling their slipping therebetween.

4. The surgical access device of any one of claims 1 to 3, wherein the one or more engagement features of the inner tube (702) and the outer tube (704) are formed around part or all of the circumference of the inner tube and the outer tube, respectively.

5. The surgical access device of claim 1,
the inner tube (702) having a distal end, a proximal end, and an opening formed therethrough and
wherein the engagement features of a first type comprise one or more ratchet features (705) disposed along part of a length of an exterior surface of the inner tube (702), the one or more ratchet features comprising one or more tooth features (705t); and
the outer tube (704) having a distal end, a proximal end, and an opening formed therethrough, the opening at the proximal end of the outer tube being configured to receive the distal end of the inner tube, and the engagement features of a second type comprising a lip feature (709) formed around the circumference of the proximal end of the outer tube (704), the lip feature (709) being configured to engage with one or more of the tooth features of the one or more ratchet features (705) of the inner tube.

6. The surgical access device of claim 5, wherein engaging the lip feature (709) of the outer tube (704) with one or more of the tooth features (705t) of the inner tube (702) causes the distal and proximal movement of the inner and outer tube, relative to each other, to be restricted.

7. The surgical access device of claim 5 or claim 6,
wherein the proximal end (702p) of the inner tube (702) is cone-shaped and flanges proximally and externally relative to the rest of the of the inner tube, and
wherein the external surface of the cone shaped portion of the inner tube is configured to contact a surface of skin of a patient's body when the surgical access device is inserted in the patient's body.

8. The surgical access device of any one of claims 5 to 7 ,
wherein a circumference of the internal surface of the outer tube (704) at the lip feature (709) is smaller than a circumference of the external surface of the inner tube (702) at the one or more tooth features.

9. The surgical access device of any one of claims 1 to 8, wherein the auxiliary tool comprises a handle portion (707) formed at a proximal end thereof, two or more prongs (710), and the at least one tab are two or more tabs (711) formed at corresponding distal ends of the two or more prongs, and the two or more prongs biased apart from one another, wherein
the one or more slots (714) of the outer tube (704) are shorter than the one or more slots of the inner tube (702), and wherein the auxiliary tool (706) is engaged with the inner and outer tubes by squeezing the two or more prongs (710) against their bias, proximally and distally moving the auxiliary tool (706) along the opening of the inner tube (702), and releasing the two or more prongs (710) causing their tabs to extend through the one or more slots (712) of the inner tube (702) and the one or more slots (714) of the outer tube (704); optionally wherein the length of the surgical access device is adjusted by proximally pulling or distally pushing the auxiliary tool (706) when the tabs (711) are extending through the one or more slots (712, 714), causing distal or proximal force to be applied on the one or more slots (714) of the outer tube (704) and the distal and proximal position of the outer tube relative to the inner tube to change as the tabs slide along the one or more slots (712) of the inner tube (702).

10. The surgical access device of any one of claims 5 to 8 and on claim 9 when dependent on any one of claims 5 to 8, wherein the proximal pulling or the distal pushing of the auxiliary tool (706) causes the lip feature (709) of the outer tube (704) to slip over one or more of the tooth features of the inner tube.

11. The surgical access device of any one of claims 1 to 4, wherein the one or more engagement features of a second type are formed by the shape of the proximal end of the outer tube (704); optionally wherein the proximal end of the outer tube (704) includes a convex portion and the one or more engagement features of a second type are formed where the convex portion of the outer tube meets a tubular portion of the outer tube (704).

12. The surgical access device of claim 1,
the inner tube (702) having a distal end and a proximal end, a hole formed therethrough from the distal end to the proximal end, and wherein the engagement features of a first type comprise one or more first ribbed features wrapped around the circumference of at least part of an external surface of the inner tube; and
the outer tube (704) having a distal end and a proximal end, a hole formed therethrough from the distal end to the proximal end, and wherein the engagement features of a second type comprise one or more second ribbed features wrapped around the circumference of at least part of an internal surface of the outer tube, the outer tube (704) having the inner tube slidably engaged therein,
wherein the one or more first ribbed features are configured to engage with the one or more second ribbed features.

13. The surgical access device of claim 12, wherein the distal and proximal positions of the inner and outer tube (702, 704), relative to each other, are fixed when the one or more first ribbed features are engaged with the one or more second ribbed features.

14. The surgical access device of claim 12 or claim 13, wherein the one or more first ribbed features and the one or more second ribbed features are convex or concave structures.

15. The surgical access device of any one of claims 12 to 14, further comprising a collar feature (910) operable to connect to a stabilization object having a fixed position relative to a body of a patient or an object.

## Patentansprüche

1. Chirurgische Zugangsvorrichtung, umfassend:
ein Innenrohr (702) mit einem oder mehreren Eingriffsmerkmalen eines ersten Typs, die an einer Außenfläche davon angeordnet sind, und wobei das Innenrohr (702) einen oder mehrere Schlitze (712) umfasst, die entlang eines Teils der Länge des Innenrohrs durch die Außenfläche und die Innenfläche des Innenrohrs (702) gebildet sind; und
ein Außenrohr (704), das teleskopartig mit dem Innenrohr (702) verbunden ist und ein oder mehrere Eingriffsmerkmale eines zweiten Typs aufweist, die mindestens an der Innenfläche davon gebildet sind, wobei das Außenrohr einen oder mehrere Schlitze (714) umfasst, die entlang eines Teils der Länge des Außenrohrs durch die Außenfläche und die Innenfläche des Außenrohrs (704) gebildet sind;
wobei der erste Typ Eingriffsmerkmal und der zweite Typ Eingriffsmerkmal komplementär zueinander sind, und
ein Hilfswerkzeug (706), das mindestens eine Nase (711) umfasst, die dazu ausgelegt ist, den mindestens einen in dem Außenrohr (704) gebildeten Schlitz (714) und den mindestens einen in dem Innenrohr (702) gebildeten Schlitz (712) in Eingriff zu nehmen, um die Ausdehnung oder das Zurückziehen des Außenrohrs (704) bezogen auf das Innenrohr (702) zu vereinfachen;
wobei eine Länge der chirurgischen Zugangsvorrichtung durch Aufbringen von entgegengesetzten proximalen und distalen Kräften auf das Innenrohr (702) und das Außenrohr (704) eingestellt wird, wodurch bewirkt wird, dass die Eingriffsmerkmale des Innenrohres von dem entsprechend einen oder den entsprechend mehreren Eingriffsmerkmalen des Außenrohres auf benachbarte andere des einen oder der mehreren Eingriffsmerkmale des Außenrohres gleiten.

2. Chirurgische Zugangsvorrichtung nach Anspruch 1, wobei das eine oder die mehreren Eingriffsmerkmale des Innenrohres (702) und des Außenrohres schräge Winkel bezogen auf eine Längsachse des Innenrohres und/oder des Außenrohres (704) bilden.

3. Chirurgische Zugangsvorrichtung nach Anspruch 1 oder Anspruch 2, wobei das eine oder die mehreren Eingriffsmerkmale des Innenrohres (702) und des Außenrohres (704) aus einem verformbaren Material gebildet sind, das ihr Gleiten dazwischen ermöglicht.

4. Chirurgische Zugangsvorrichtung nach einem der Ansprüche 1 bis 3, wobei das eine oder die mehreren Eingriffsmerkmale des Innenrohres (702) und des Außenrohres (704) um einen Teil des Umfangs oder um den ganzen Umfang des Innenrohres bzw. des Außenrohres gebildet sind.

5. Chirurgische Zugangsvorrichtung nach Anspruch 1,
wobei das Innenrohr (702) ein distales Ende, ein proximales Ende und eine Öffnung hat, die dort hindurch gebildet ist, und wobei die Eingriffsmerkmale eines ersten Typs ein oder mehrere Ratschenmerkmale (705) umfasst, die an einem Teil einer Länge einer Außenfläche des Innenrohres (702) angeordnet sind, wobei das eine oder die mehreren Ratschenmerkmale ein oder mehrere Zahnmerkmale (705t) umfassen; und
wobei das Außenrohr (704) ein distales Ende, ein proximales Ende und eine Öffnung hat, die dort hindurch gebildet ist, wobei die Öffnung an dem proximalen Ende des Außenrohres dazu ausgelegt ist, das distale Ende des Innenrohres aufzunehmen, und wobei die Eingriffsmerkmale eines zweiten Typs ein Lippenmerkmal (709) umfassen, das um den Umfang des proximalen Endes des Außenrohres (704) gebildet ist, wobei das Lippenmerkmal (709) dazu ausgelegt ist, eins oder mehrere der Zahnmerkmale des einen oder der mehreren Ratschenmerkmale (705) des Innenrohres in Eingriff zu nehmen.

6. Chirurgische Zugangsvorrichtung nach Anspruch 5, wobei das Eingreifen des Lippenmerkmals (709) des Außenrohres (704) in eins oder mehrere der Zahnmerkmale (705t) des Innenrohres (702) bewirkt, dass die distale und die proximale Bewegung des Innen- und des Außenrohres bezogen aufeinander, eingeschränkt wird.

7. Chirurgische Zugangsvorrichtung nach Anspruch 5 oder Anspruch 6,
wobei das proximale Ende (702p) des Innenrohres (702) kegelförmig ist und bezogen auf den Rest des Innenrohres proximal und nach außen flanscht, und
wobei die Außenfläche des kegelförmigen Abschnitts des Innenrohres dazu ausgelegt ist, eine Oberfläche der Haut des Körpers eines Patienten zu berühren, wenn die chirurgische Zugangsvorrichtung in den Körper des Patienten eingesetzt wird.

8. Chirurgische Zugangsvorrichtung nach einem der Ansprüche 5 bis 7,
wobei ein Umfang der Innenfläche des Außenrohres (704) an dem Lippenmerkmal (709) kleiner ist als ein Umfang der Außenfläche des Innenrohres (702) an dem einen oder den mehreren Zahnmerkmalen.

9. Chirurgische Zugangsvorrichtung nach einem der Ansprüche 1 bis 8, wobei das Hilfswerkzeug einen Griffabschnitt (707), der an einem proximalen Ende davon gebildet ist und zwei oder mehrere Schenkel (710) umfasst und die mindestens eine Nase zwei oder mehrere Nasen (711) sind, die an entsprechenden distalen Enden der zwei oder mehreren Schenkel gebildet sind, und die zwei oder mehreren Schenkel voneinander weg vorgespannt sind, wobei der eine oder die mehreren Schlitze (714) des Außenrohres (704) kürzer sind als der eine oder die mehreren Schlitze des Innenrohres (702), und wobei das Hilfswerkzeug (706) durch Zusammendrücken der zwei oder mehreren Schenkel (710) gegen ihre Vorspannung, proximales und distales Bewegen des Hilfswerkzeugs (706) entlang der Öffnung des Innenrohres (702) und Freigeben der zwei oder mehreren Schenkel (710) mit dem Innen- und dem Außenrohr in Eingriff gebracht wird, was bewirkt, dass sich ihre Nasen durch den einen oder die mehreren Schlitze (712) des Innenrohres (702) und den einen oder die mehreren Schlitze (714) des Außenrohres (704) erstrecken; wobei optional die Länge der chirurgischen Zugangsvorrichtung durch proximales Ziehen oder distales Drücken des Hilfswerkzeugs (706) eingestellt wird, wenn sich die Nasen (711) durch den einen oder die mehreren Schlitze (712, 714) erstrecken, was bewirkt, dass eine distale oder proximale Kraft auf den einen oder die mehreren Schlitze (714) des Außenrohres (704) aufgebracht wird und sich die distale und die proximale Position des Außenrohres bezogen auf das Innenrohr ändern, wenn die Nasen den einen oder die mehreren Schlitze (712) des Innenrohres (702) entlang gleiten.

10. Chirurgische Zugangsvorrichtung nach einem der Ansprüche 5 bis 8 und nach Anspruch 9, wenn von einem der Ansprüche 5 bis 8 abhängig, wobei das proximale Ziehen oder das distale Drücken des Hilfswerkzeugs (706) bewirkt, dass das Lippenmerkmal (709) des Außenrohres (704) über eins oder mehrere der Zahnmerkmale des Innenrohres gleitet.

11. Chirurgische Zugangsvorrichtung nach einem der Ansprüche 1 bis 4, wobei das eine oder die mehreren Eingriffsmerkmale eines zweiten Typs durch die Form des proximalen Endes des Außenrohres (704) gebildet sind; wobei optional das proximale Ende des Außenrohres (704) einen konvexen Abschnitt aufweist und das eine oder die mehreren Eingriffsmerkmale eines zweiten Typs dort gebildet sind, wo der konvexe Abschnitt des Außenrohres auf einen röhrenförmigen Abschnitt des Außenrohres (704) trifft.

12. Chirurgische Zugangsvorrichtung nach Anspruch 1,
wobei das Innenrohr (702) ein distales Ende und ein proximales Ende hat, ein Loch, das von dem distalen Ende zu dem proximalen Ende dort hindurch gebildet ist, und wobei die Eingriffsmerkmale eines ersten Typs ein oder mehrere erste gerippte Merkmale umfassen, die um den Umfang von mindestens einem Teil einer Außenfläche des Innenrohres gewickelt sind; und
wobei das Außenrohr (704) ein distales Ende und ein proximales Ende hat, ein Loch, das von dem distalen Ende zu dem proximalen Ende dort hindurch gebildet ist, und wobei die Eingriffsmerkmale eines zweiten Typs ein oder mehrere zweite gerippte Merkmale umfassen, die um den Umfang von mindestens einem Teil einer Innenfläche des Außenrohres gewickelt sind, wobei das Außenrohr (704) das Innenrohr darin verschiebbar im Eingriff hat,
wobei das eine oder die mehreren ersten gerippten Merkmale dazu ausgelegt sind, das eine oder die mehreren zweiten gerippten Merkmale in Eingriff zu nehmen.

13. Chirurgische Zugangsvorrichtung nach Anspruch 12, wobei die distale und die proximale Position des Innen- und des Außenrohres (702, 704), bezogen aufeinander, fest sind, wenn das eine oder die mehreren ersten gerippten Merkmale mit dem einen oder den mehreren zweiten gerippten Merkmalen im Eingriff sind.

14. Chirurgische Zugangsvorrichtung nach Anspruch 12 oder Anspruch 13, wobei das eine oder die mehreren ersten gerippten Merkmale und das eine oder die mehreren zweiten gerippten Merkmale konvexe oder konkave Strukturen sind.

15. Chirurgische Zugangsvorrichtung nach einem der Ansprüche 12 bis 14, ferner umfassend ein Kragenmerkmal (910), das bedienbar ist, um sich mit einem Stabilisierungsobjekt zu verbinden, das eine feste Position bezogen auf einen Körper eines Patienten oder eines Objekts hat.

## Revendications

1. Dispositif d'accès chirurgical comprenant :
un tube intérieur (702) comprenant une ou plusieurs caractéristiques de mise en prise d'un premier type disposées sur sa surface extérieure, et le tube intérieur (702) comprenant une ou plusieurs fentes (712) formées le long d'une partie de la longueur du tube intérieur, à travers les surfaces externe et interne du tube intérieur (702) ; et
un tube extérieur (704) accouplé de manière télescopique au tube intérieur (702), et comprenant une ou plusieurs caractéristiques de mise en prise d'un second type formées au moins sur sa surface interne, le tube extérieur comprenant une ou plusieurs fentes (714) formées le long d'une partie de la longueur du tube extérieur, à travers les surfaces externe et interne du tube extérieur (704) ;
le premier type de caractéristiques de mise en prise et le second type de caractéristiques de mise en prise étant complémentaires l'un de l'autre, et
un outil auxiliaire (706) comprenant au moins une languette (711) configurée pour venir en prise avec au moins une fente (714) formée dans le tube extérieur (704) et au moins une fente (712) formée dans le tube intérieur (702) pour faciliter l'extension ou la rétraction du tube extérieur (704) par rapport au tube intérieur (702) ;
une longueur du dispositif d'accès chirurgical étant ajustée en appliquant des forces proximales ou distales opposées sur le tube intérieur (702) et le tube extérieur (704), provoquant ainsi le glissement des caractéristiques de mise en prise du tube intérieur depuis la ou les caractéristiques de mise en prise correspondantes du tube extérieur vers d'autres caractéristiques de mise en prise adjacentes de la ou des caractéristiques de mise en prise du tube extérieur.

2. Dispositif d'accès chirurgical selon la revendication 1, la ou les caractéristiques de mise en prise du tube intérieur (702) et du tube extérieur formant des angles obliques par rapport à un axe longitudinal du tube intérieur et/ou du tube extérieur (704).

3. Dispositif d'accès chirurgical selon la revendication 1 ou selon la revendication 2, la ou les caractéristiques de mise en prise du tube intérieur (702) et du tube extérieur (704) étant formées d'un matériau déformable permettant leur glissement entre eux.

4. Dispositif d'accès chirurgical selon l'une quelconque des revendications 1 à 3, la ou les caractéristiques de mise en prise du tube intérieur (702) et du tube extérieur (704) étant formées respectivement autour d'une partie ou de la totalité de la circonférence du tube intérieur et du tube extérieur.

5. Dispositif d'accès chirurgical selon la revendication 1,
le tube intérieur (702) ayant une extrémité distale, une extrémité proximale et une ouverture formée à travers lui et les caractéristiques de mise en prise d'un premier type comprenant une ou plusieurs caractéristiques de cliquet (705) disposées le long d'une partie de la longueur d'une surface extérieure du tube intérieur (702), la ou les caractéristiques de cliquet comprenant une ou plusieurs caractéristiques de dent (705t) ; et
le tube extérieur (704) ayant une extrémité distale, une extrémité proximale et une ouverture formée à travers lui, l'ouverture à l'extrémité proximale du tube extérieur étant configurée pour recevoir l'extrémité distale du tube intérieur, et les caractéristiques de mise en prise d'un second type comprenant une caractéristique de lèvre (709) formée autour de la circonférence de l'extrémité proximale du tube extérieur (704), la caractéristique de lèvre (709) étant configurée pour venir en prise avec une ou plusieurs des caractéristiques de dent de la ou des caractéristiques de cliquet (705) du tube intérieur.

6. Dispositif d'accès chirurgical selon la revendication 5, la mise en prise de la caractéristique de lèvre (709) du tube extérieur (704) avec une ou plusieurs des caractéristiques de dent (705t) du tube intérieur (702) entraînant la restriction du mouvement distal et proximal des tubes intérieur et extérieur, l'un par rapport à l'autre.

7. Dispositif d'accès chirurgical selon la revendication 5 ou selon la revendication 6,
l'extrémité proximale (702p) du tube intérieur (702) étant en forme de cône et s'évasant proximalement et extérieurement par rapport au reste du tube intérieur, et
la surface externe de la partie en forme de cône du tube intérieur étant configurée pour entrer en contact avec une surface de peau du corps d'un patient lorsque le dispositif d'accès chirurgical est inséré dans le corps du patient.

8. Dispositif d'accès chirurgical selon l'une quelconque des revendications 5 à 7,
une circonférence de la surface interne du tube extérieur (704) au niveau de la caractéristique de lèvre (709) étant inférieure à la circonférence de la surface externe du tube intérieur (702) au niveau d'une ou plusieurs caractéristiques de dent.

9. Dispositif d'accès chirurgical selon l'une quelconque des revendications 1 à 8, l'outil auxiliaire comprenant une partie poignée (707) formée à une extrémité proximale de celui-ci, deux ou plus de deux broches (710), et l'au moins une languette étant deux ou plus de deux languettes (711) formées à des extrémités distales correspondantes des deux ou plus de deux broches, et les deux ou plus de deux broches étant écartées l'une de l'autre,
la ou les fentes (714) du tube extérieur (704) étant plus courtes que la ou les fentes du tube intérieur (702), et l'outil auxiliaire (706) venant en prise avec les tubes intérieur et extérieur en pressant les deux ou plus de deux broches (710) contre leur écartement, en déplaçant proximalement et distalement l'outil auxiliaire (706) le long de l'ouverture du tube intérieur (702), et en relâchant les deux ou plus de deux broches (710) pour que leurs languettes s'étendent à travers la ou les fentes (712) du tube intérieur (702) et la ou les fentes (714) du tube extérieur (704) ; éventuellement, la longueur du dispositif d'accès chirurgical étant ajustée en tirant proximalement ou en poussant distalement l'outil auxiliaire (706) lorsque les languettes (711) s'étendent à travers la ou les fentes (712, 714), ce qui entraîne l'application d'une force distale ou proximale sur la ou les fentes (714) du tube extérieur (704) et la modification de la position distale et proximale du tube extérieur par rapport au tube intérieur à mesure que les languettes glissent le long de la ou des fentes (712) du tube intérieur (702).

10. Dispositif d'accès chirurgical selon l'une quelconque des revendications 5 à 8 et selon la revendication 9 lorsqu'elle dépend de l'une quelconque des revendications 5 à 8, la traction proximale ou la poussée distale de l'outil auxiliaire (706) faisant glisser la caractéristique de lèvre (709) du tube extérieur (704) sur une ou plusieurs des caractéristiques de dent du tube intérieur.

11. Dispositif d'accès chirurgical selon l'une quelconque des revendications 1 à 4, la ou les caractéristiques de mise en prise d'un second type étant formées par la forme de l'extrémité proximale du tube extérieur (704) ; éventuellement, l'extrémité proximale du tube extérieur (704) comprenant une partie convexe et la ou les caractéristiques de mise en prise d'un second type étant formées à l'endroit où la partie convexe du tube extérieur rencontre une partie tubulaire du tube extérieur (704).

12. Dispositif d'accès chirurgical selon la revendication 1,
le tube intérieur (702) ayant une extrémité distale et une extrémité proximale, un trou formé à travers celui-ci de l'extrémité distale à l'extrémité proximale, et les caractéristiques de mise en prise d'un premier type comprenant une ou plusieurs premières caractéristiques nervurées enveloppant la circonférence d'au moins une partie d'une surface externe du tube intérieur ; et
le tube extérieur (704) ayant une extrémité distale et une extrémité proximale, un trou formé à travers celui-ci de l'extrémité distale à l'extrémité proximale, et les caractéristiques de mise en prise d'un second type comprenant une ou plusieurs secondes caractéristiques nervurées enveloppant la circonférence d'au moins une partie d'une surface interne du tube extérieur, le tube extérieur (704) ayant le tube intérieur en prise de manière coulissante à l'intérieur de celui-ci,
la ou les premières caractéristiques nervurées étant configurées pour venir en prise avec la ou les secondes caractéristiques nervurées.

13. Dispositif d'accès chirurgical selon la revendication 12, les positions distale et proximale des tubes intérieur et extérieur (702, 704), l'une par rapport à l'autre, étant fixes lorsque la ou les premières caractéristiques nervurées sont en prise avec la ou les secondes caractéristiques nervurées.

14. Dispositif d'accès chirurgical selon la revendication 12 ou selon la revendication 13, la ou les premières caractéristiques nervurées et la ou les secondes caractéristiques nervurées étant des structures convexes ou concaves.

15. Dispositif d'accès chirurgical selon l'une quelconque des revendications 12 à 14, comprenant en outre une caractéristique de collier (910) utilisable pour se connecter à un objet de stabilisation ayant une position fixe par rapport au corps d'un patient ou d'un objet.
